# EUROPEAN PATENT APPLICATION

(11) **EP 4 455 143 A1**
(43) Date of publication of application: **30.10.2024**
(21) Application number: 22911817.9
(22) Date of filing: 19.12.2022
(51) Int. Cl.: C07D 417/06, A61K 31/4439, A61P 35/00

(54) **SUBSTITUTED THIAZOLIDINEDIONE DERIVATIVE COMPOUND, AND PHARMACEUTICAL COMPOSITION FOR PREVENTING OR TREATING CANCER, COMPRISING SAME**

(30) Priority: 21.12.2021 KR 20210183638; 15.12.2022 KR 20220175867
(71) Applicant: Medific, Inc., Hwaseong-si, Gyeonggi-do 18469 (KR)
(72) Inventor: KIM, Jun-Kyum, Hwaseong-si Gyeonggi-do 18447 (KR); CHOI, Jia, Yongin-si Gyeonggi-do 16833 (KR); KIM, Eun-Jung, Hwaseong-si Gyeonggi-do 18478 (KR); PARK, Cheol-Kyu, Hwaseong-si Gyeonggi-do 18429 (KR); HAM, Seok Won, Hwaseong-si Gyeonggi-do 18466 (KR); PARK, Min Gi, Hwaseong-si Gyeonggi-do 18478 (KR); JEONG, Hyeon Ju, Hwaseong-si Gyeonggi-do 18479 (KR); KIM, Sung Jin, Hwaseong-si Gyeonggi-do 18479 (KR); MIN, Kyungim, Hwaseong-si Gyeonggi-do 18420 (KR); PARK, Jong Min, Hwaseong-si Gyeonggi-do 18469 (KR); CHIN, Jungwook, Daegu 41061 (KR); CHO, Sung Jin, Daegu 41061 (KR); KIM, Jina, Daegu 41061 (KR); JUNG, Kyung Jin, Daegu 41061 (KR); KIM, Nayeon, Daegu 41061 (KR); KIM, Suhui, Daegu 41061 (KR); KWON, Sugyeong, Daegu 41061 (KR); LEE, Su-Jeong, Hwaseong-si Gyeonggi-do 18454 (KR); JEONG, Minseon, Daegu 41061 (KR); AN, Hongchan, Daegu 41061 (KR); PARK, Jeong-Eun, Daegu 41061 (KR); KIM, Dong-Hyun, Daegu 41061 (KR); LIM, Ji-youn, Daegu 41061 (KR); MIN, Ju-sik, Daegu 41061 (KR); HWANG, Ji Sun, Daegu 41061 (KR); CHOI, Hyo-Jung, Daegu 41061 (KR); HWANG, Hayoung, Daegu 41061 (KR); KWON, Oh-Bin, Daegu 41061 (KR); LEE, Sungwoo, Daegu 41061 (KR); KIM, Sang Bum, Daegu 41061 (KR)
(74) Representative: Kraus & Lederer PartGmbB
(86) International application number: PCT/KR2022/020728
(87) International publication number: WO 2023/121184

(57) **Abstract**

The present invention relates to: a substituted thiazolidinedione derivative compound having a novel structure acting as a sterol regulatory element-binding protein-1 (SREBP1) inhibitor, a hydrate thereof, or a pharmaceutically acceptable salt thereof; and a pharmaceutical composition for preventing or treating cancer, comprising same as an active ingredient.

## Description

### [Technical Field]

The present invention relates to a substituted thiazolidinedione derivative compound having a novel structure which acts as a sterol regulatory element-binding protein-1 (SREBP1) inhibitor, a hydrate thereof, or a pharmaceutically acceptable salt thereof, and a pharmaceutical composition for preventing or treating cancer including the compound as an effective component.

### [Background Art]

Sterol regulatory element-binding protein-1 (SREBP1) is an important transcription factor in fat synthesis and metabolism. It precisely regulates the expression of enzymes required for the synthesis of enzyme endogenous cholesterol, fatty acid (FA), triacylglycerol, and/or phospholipid.

Recently, it has been reported in many works of literature that SREBP1 plays a very important linking role in cancer metabolism and tumor signaling. Recently, according to research results, it was reported that SREBP1 is required for the survival of brain tumor cells having an epidermal growth factor (EGF) signal-dependent anticancer agent resistance mechanism, and it was confirmed in the research that SREBP1 is dependent on an increase in lowdensity lipoprotein receptor (LDLR) and fatty acid synthesis. As a result, it is known that survival and proliferation of brain tumor cells are promoted through EGFR/PI3K/Akt/SREBP1 signal transduction pathways.

A brain tumor refers collectively to primary brain cancer, which occurs in brain tissues and the meninges surrounding the brain, and secondary brain cancer, which metastases from cancer originating in the skull or other parts of the body and brain cancer is different from cancer occurring in other organ in many ways. Cancer occurring in the lungs, stomach, breasts, and the like is limited to one or two types per organ, and the nature is the same or similar. Still, a wide variety of cancers such as glioblastoma multiforme, malignant glioma, lymphoma, germ cell tumor, and a metastatic tumor occur in the brain.

A brain tumor found in adults includes glioma, glioblastoma, meningioma, pituitary adenoma, schwannoma, and the like. Among them, glioblastoma is a tumor starting from glial cells of brain tissue, accounts for 12 to 15% of all brain tumors, and is known as most common primary brain tumor in adults. The brain tumor is a representative intractable solid tumor of which the improvement in survival rate due to treatment is insignificant, and its fundamental therapeutic effect may not be derived from a conventional cancer treatment method.

Thus, the development of an inhibitor that may selectively inhibit SREBP1, which plays a very important linking role in cancer metabolism and tumor signaling, is demanded.

### [Disclosure]

### [Technical Problem]

Thus, the present inventors synthesized a compound having a novel structure that may act as an inhibitor against SREBP1 and confirmed that the compound shows an anticancer effect in cancer, in particular, a brain tumor model in vivo and in vitro, and may be used as a new anticancer agent, thereby completing the present invention.

The object of the present invention is to provide a substituted thiazolidinedione derivative compound with a novel structure that may act as an SREBP1 inhibitor, a hydrate thereof, or a pharmaceutically acceptable salt thereof.

Another object of the present invention is to provide a pharmaceutical composition for preventing or treating cancer, including the substituted thiazolidinedione derivative compound, a hydrate thereof, or a pharmaceutically acceptable salt thereof as an effective component.

Another object of the present invention is to provide a health functional food composition for preventing or ameliorating cancer, including the substituted thiazolidinedione derivative compound, a hydrate thereof, or a pharmaceutically acceptable salt thereof as an effective component.

Another object of the present invention is to provide a method for treating cancer, including administrating the substituted thiazolidinedione derivative compound, a hydrate thereof, or a pharmaceutically acceptable salt thereof to an individual or subject in need thereof.

Another object of the present invention is to provide the substituted thiazolidinedione derivative compound, the hydrate thereof, or the pharmaceutically acceptable salt thereof for use in treatment of cancer.

Still another object of the present invention is to provide a use of the substituted thiazolidinedione derivative compound, the hydrate thereof, or the pharmaceutically acceptable salt thereof for use in preparation of a pharmaceutical agent for treating cancer.

### [Technical Solution]

In order to achieve the above objects,

in one general aspect, a substituted thiazolidinedione derivative compound represented by the following Chemical Formula 1, a hydrate thereof, or a pharmaceutically acceptable salt thereof is provided:

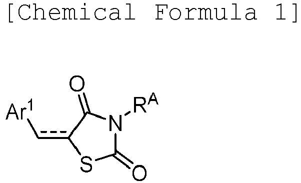

wherein
-̅ -̅ -̅ is a single bond or double bond;
Ar¹ is 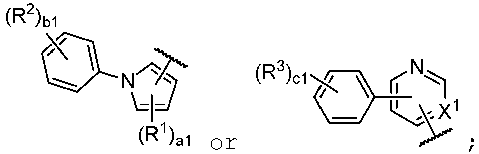
R¹ is C1-C10alkyl;
R² is haloC1-C10alkyloxy or C6-C12arylC1-C10alkyloxy;
R³ is C1-C10alkyl, haloC1-C10alkyl, C1-C10alkoxy, haloC1-C10alkoxy, or C6-C12arylalkyloxy;
X¹ is CH or N;
R^{A} is hydrogen, C1-C10alkyl, C3-C10cycloalkyl, C6-C12aryl, C6-C12arylC1-C10alkyl, or -L¹-Het¹;
L¹ is C1-C10alkylene;
Het¹ is C3-C10heterocycloalkyl;
a1 is an integer of 0 to 3; and
b1 and c1 are independently of each other an integer of 0 to 5.

In another general aspect, a pharmaceutical composition for preventing or treating cancer includes a substituted thiazolidinedione derivative compound represented by the following Chemical Formula 2, a hydrate thereof, or a pharmaceutically acceptable salt thereof as an effective component:

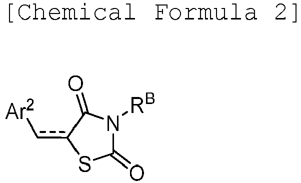

wherein
-̅ -̅ -̅ is a single bond or double bond;
Ar² is 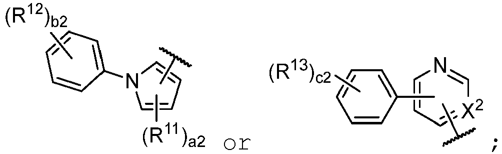
R¹¹ is C1-C10alkyl;
R¹² is C1-C10alkyl, hydroxy, haloC1-C10alkyloxy, or C6-C12arylC1-C10alkyloxy;
R¹³ is C1-C10alkyl, haloC1-C10alkyl, C1-C10alkoxy, haloC1-C10alkoxy, or C6-C12arylalkyloxy;
X² is CH or N;
R^{B} is hydrogen, C1-C10alkyl, C3-C10cycloalkyl, C6-C12aryl, C6-C12arylC1-C10 alkyl, or -L²-Het²;
L² is C1-C10alkylene;
Het² is C3-C10heterocycloalkyl;
a2 is an integer of 0 to 3; and
b2 and c2 are independent of each other and are integers of 0 to 5.

In another general aspect, a health functional food composition includes a substituted thiazolidinedione derivative compound represented by Chemical Formula 2, a hydrate thereof, or a sitologically acceptable salt thereof as an effective component.

In another general aspect, a method for treating cancer includes administrating the substituted thiazolidinedione derivative compound represented by Chemical Formula 2, the hydrate thereof, or the pharmaceutically acceptable salt thereof to an individual or subject in need thereof.

In another general aspect, the substituted thiazolidinedione derivative compound represented by Chemical Formula 2, the hydrate thereof, or the pharmaceutically acceptable salt thereof for use in cancer treatment is provided.

Another general aspect is the use of the substituted thiazolidinedione derivative compound represented by Chemical Formula 2, the hydrate thereof, or the pharmaceutically acceptable salt thereof for use in preparation of a pharmaceutical agent for treating cancer.

### [Advantageous Effects]

The present disclosure relates to a novel substituted thiazolidinedione derivative compound and a pharmaceutical composition for preventing or treating cancer, including the compound as an effective component, and since the compound, according to an exemplary embodiment, has high biocompatibility and high selectivity and maybe act as an effective SREBP1 inhibitor which is orally bioavailable, it may be useful as a pharmaceutical composition for preventing or treating cancer.

Since the compound, according to an exemplary embodiment, acts as a SREBP1 inhibitor without showing toxicity to normal cells to specifically induce apoptosis in cancer cell lines, in particular, a brain tumor, it may be useful as an agent for efficiently ameliorating, preventing, and treating a brain tumor having an epidermal growth factor (EGF) signal-dependent anticancer agent resistance mechanism without side effects.

### [Description of Drawings]

FIG. 1 shows the measurement of the activity of derivatives using a cell-based assay system [(a) a cell-based assay system using a 6xSRE reporter vector; (b) and (c) changes in expression level and GFP expression amount of SREBP1a and SREBP1c in an activated form in separated GFP-low cells; and (d) changes in GFP expression amount when treated with each of fatostatin and FGH10019 which are known to inhibit DMSO and SREBP activity as a control group].
FIG. 2 shows a comparison experiment of cell viability depending on compound treatment in brain tumor stem cell HOG-GSC.
FIG. 3 shows results of Western blot assay of Compound 11 (Example 11).
FIG. 4 is a graph showing the cell viability of brain tumor stem cell HOG-GSC depending on the concentration of Compound 11 (Example 11).
FIG. 5 is a graph showing drug concentration in plasma after oral administration (25 mg/kg).
FIG. 6 is a graph showing drug concentration in brain tissue after oral administration (25 mg/kg).
FIG. 7 is a schematic diagram for an in-vivo efficacy evaluation test.
FIG. 8 is a graph showing a survival rate over time.
FIG. 9 is a photograph of the brain tissue of a mouse that finally survived on day 66 after tumor transplantation.
FIG. 10 is a photograph of the brain tissue of a mouse that survived on day 16 after tumor transplantation.

### [Best Mode]

Hereinafter, the present invention will be described in detail. Here, technical terms and scientific terms used in the present specification have the general meaning understood by those skilled in the art to which the present invention pertains unless otherwise defined, and description for the known function and configuration which may unnecessarily obscure the gist of the present invention will be omitted in the following description.

The embodiments described in the present specification may be modified in many different forms, and the technology according to one embodiment is not limited to the embodiments set forth herein. In addition, the embodiments of an exemplary embodiment are provided so that the present invention will be described in more detail to a person with ordinary skill in the art. Furthermore, throughout the specification, unless explicitly described to the contrary, "comprising" any constituent elements will be understood to imply further inclusion of other constituent elements rather than exclusion of other constituent elements.

The numerical range used in the present specification includes all values within the range, including the lower limit and the upper limit, increments logically derived in a form and spanning in a defined range, all double limited values, and all possible combinations of the upper limit and the lower limit in the numerical range defined in different forms. As an example, when it is defined that a content of a composition is 10% to 80% or 20% to 50%, it should be interpreted that a numerical range of 10% to 50% or 50% to 80% is also described in the specification of the present. Unless otherwise defined in the present specification, values that may be outside a numerical range due to experimental error or rounding off of a value are also included in the defined numerical range.

Hereinafter, unless otherwise particularly defined in the present specification, "about" may be considered as a value within 30%, 25%, 20%, 15%, 10%, or 5% of a stated value.

The following terms used in the present specification are defined as follows, but they are only illustrative and do not limit the present invention, application, or use.

The singular form used in the present specification may be intended also to include a plural form unless otherwise indicated in the context.

The terms "substitution group or substituent", "radical", "group", "moiety", and "fragment" in the present specification may be used interchangeably.

The term "C_{A}-C_{B}" in the present specification refers to "having A or more and B or fewer carbon atoms", and the term "A to B" refers to "A or more and B or less".

The term "hydrocarbyl" refers to a radical having one bonding site derived from hydrocarbon, and the term "aromatic hydrocarbyl" refers to a radical having one bonding site derived from an aromatic hydrocarbon compound in the present specification.

In the description of "unsubstituted or substituted" in the present specification, "substituted" refers to a group or site having one or more substituents attached to a structural skeleton of a group or a portion. It means substituted on a non-limitingly mentioned group or structural skeleton by a group other than hydrogen.

The term "alkyl" in the present specification refers to a monovalent straight chain or branched chain saturated hydrocarbon radical formed of only carbon and hydrogen atoms. The alkyl may have 1 to 10, 1 to 7, or 1 to 4 carbon atoms. As an example, the alkyl includes methyl, ethyl, propyl, isopropyl, butyl, isobutyl, t-butyl, pentyl, hexyl, ethylhexyl, and the like, but is not limited thereto.

The term "cycloalkyl" in the present specification refers to a monovalent saturated carbocyclic radical formed of a monocycle. An example of the cycloalkyl radical includes cyclopropyl, cyclobutyl, cyclopentyl, cyclohexyl, cycloheptyl, and the like, but is not limited thereto.

The term "alkoxy" in the present specification is a - O-alkyl radical, in which "alkyl" is as defined above. A specific example thereof includes methoxy, ethoxy, isopropoxy, butoxy, isobutoxy, t-butoxy, and the like, but is not limited thereto.

The term "halo" or "halogen" in the present specification refers to a halogen group element, and for example, includes fluoro, chloro, bromo, and iodo.

The term "haloalkyl" or "haloalkoxy" in the present specification refers to an alkyl or alkoxy group in which one or more hydrogen atoms are substituted with a halogen atom, respectively, wherein alkyl, alkoxy, and halogen are as defined above. For example, haloalky may be fluoromethyl, difluoromethyl, trifluoromethyl, fluoroethyl, difluoroethyl, perfluoroethyl, and the like, and haloalkoxy may be fluoromethoxy, difluoromethoxy, trifluoromethoxy, fluoroethoxy, difluoroethoxy, perfluoroethoxy, and the like.

The term "aryl" in the present specification refers to an organic radical derived from an aromatic hydrocarbon by removal of one hydrogen, and includes a monocyclic or fused ring system containing suitably 4 to 7, preferably 5 or 6 ring atoms in each ring, and even a form in which a plurality of aryls are connected by a single bond. As an example, aryl includes phenyl, naphthyl, biphenyl, terphenyl, anthryl, fluorenyl, and the like, but is not limited thereto.

The term "heterocycloalkyl" in the present specification is a monovalent radical of a saturated heterocycle containing 1 to 4 heteroatoms selected from N, O, and S, and the saturated heterocycle includes not only monocycle, polycycles, or spirocycle forms but also a form fused with an aromatic hydrocarbon ring such as benzene, and may be bonded through a heteroatom or carbon atom. In addition, the carbon atom in the saturated heterocycle may be substituted with oxo. An example of the heterocycloalkyl radical may include monovalent radicals of a saturated heterocycle such as aziridine, oxetane, pyrrolidine, tetrahydrofuran, dioxolane, dithiolane, imidazolidine, tetrahydropyran, pyrazolidine, pyrrolidinone, azetidine, piperidine, piperazine, morpholine, thiomorpholine, dioxane, hexahydroazepine, thiolane, dihydroisoindole, benzodioxole, dihydrobenzodioxine, isoindolinone, indolinone, dihydroisoquinolinone, tetrahydrobenzoazepinone, 3-azabicyclo[3.1.0]hexane, octahydropyrrolo[3,4-c]pyrrole, 2,7-diazaspiro[4.4]nonane, and 2-azaspiro[4.4]nonane.

The term "pharmaceutically acceptable" in the present specification, which shows non-toxic properties to individuals such as cells or humans exposed to the composition, means that it is appropriate for use as a pharmaceutical preparation, is generally regarded as being safe for the use, and means that it is officially approved by the national management agency or listed in the Korean Pharmacopoeia or the US Pharmacopoeia.

The term "pharmaceutically acceptable salt" in the present specification refers to any organic or inorganic addition salt of the compound of the present invention at a concentration having a relatively non-toxic and harmless effective action to a patient, which does not reduce advantageous efficacy of the compound of the present invention itself by the side effect caused by the salt.

The terms "pharmaceutically acceptable excipient" and "pharmaceutically acceptable carrier" in the present specification refer to materials which help administration of an activator and absorption by a subject.

The term "prevention" in the present specification refers to all actions of inhibiting or delaying the occurrence, spread, and recurrence of cancer.

The term "amelioration" refers to all actions of at least decreasing parameters related to the conditions to be treated, for example, the severity of symptoms.

The term "treatment" in the present specification refers to all actions of ameliorating or beneficially altering the symptoms of cancer.

The term "individual" in the present specification refers to all animals, including humans, who have or are likely to develop cancer. The animals may be mammals such as cattle, horses, sheep, pigs, goats, camels, antelopes, dogs, and cats in need of treatment of similar symptoms, as well as humans, but are not limited thereto.

The term "administration" in the present specification means that the pharmaceutical composition of the present invention is introduced to an individual by any appropriate method, and the administration route of the composition of the present invention may be various, such as oral or parenteral, as long as the composition may reach a target tissue.

The term "pharmaceutically effective amount" in the present specification refers to an amount which is sufficient to treat a disease at a reasonable benefit/risk ratio applicable to medical treatment and does not cause side effects, and an effective dose level may be easily determined by a person skilled in the art according to factors including patients' gender, age, weight, and health state, the kind of diseases, severity, an activity of a drug, a sensitivity to a drug, an administration manner, an administration time, administration route and releasing rate, a treatment period, formulation, or a simultaneously used drug, and other factors well known in the medical field.

The term "food" in the present specification may be meat, sausage, bread, chocolate, candy, snacks, confectionery, pizza, ramen, other noodles, chewing gum, dairy products including ice cream, various soups, beverages, tea, drinks, alcoholic beverages, vitamin complexes, health functional food, health food, and the like, and includes all food in common sense.

The term "health functional food" in the present specification refers to food manufactured and processed using raw materials or components having functionality useful to the human body in accordance with Functional Foods for Health Act No. 6727, and "functional" refers to intake for the purpose of regulating nutrients for the structure and function of the human body or obtaining a beneficial effect for health applications such as physiological action.

The term "sitologically acceptable salt" in the present specification refers to a formulation of a compound, which does not cause serious irritation to an organism to which a compound is administered and does not damage the biological activity and physical properties of the compound.

An exemplary embodiment provides a substituted thiazolidinedione derivative compound represented by the following Chemical Formula 1, a hydrate thereof, or a pharmaceutically acceptable salt thereof:

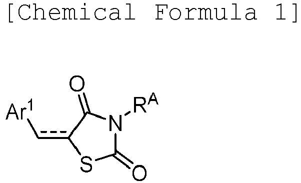

wherein
-̅ -̅ -̅ is a single bond or double bond;
Ar¹ is 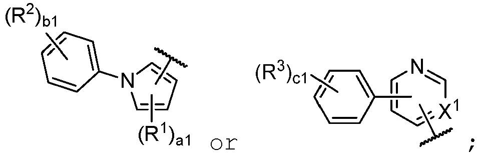
R₁ is C1-C10alkyl;
R² is haloC1-C10alkyloxy or C6-C12arylC1-C10alkyloxy;
R³ is C1-C10alkyl, haloC1-C10alkyl, C1-C10alkoxy, haloC1-C10alkoxy, or C6-C12arylalkyloxy;
X¹ is CH or N;
R^{A} is hydrogen, C1-C10alkyl, C3-C10cycloalkyl, C6-C12aryl, C6-C12arylC1-C10alkyl, or -L¹-Het¹;
L¹ is C1-C10alkylene;
Het¹ is C3-C10heterocycloalkyl;
a1 is an integer of 0 to 3; and
b1 and c1 are independently of each other an integer of 0 to 5.

The substituted thiazolidinedione derivative compound of Chemical Formula 1 according to the present invention is a compound having a novel structure, and inhibits SREBP1 essential for survival of cancer stem cells and may be used as an effective component which prevents of treats cancer. The cancer stem cells which cause growth and metastasis of cancer cells and anticancer agent resistance is highly dependent on lipid metabolism regulation by SREBP1 which is a transcription factor protein promoting lipid synthesis. Therefore, since the compound according to the present invention acts as a SREBP1 inhibitor and selectively inhibits SREBP1 to remove cancer stem cells and cancer cell, it may be useful as a targeted anticancer agent of cancer.

In addition, the compound of the present invention has high biocompatibility and high selectivity, may be orally bioavailable, and may act as a SREBP1 inhibitor to specifically induce apoptosis only to cancer cell lines without showing toxicity to normal cells, it may be useful as an efficient agent for preventing and treating cancer without side effects. Cancer diseases which may be prevented, treated, or ameliorated by the treatment with the compound according to the present invention are intractable solid cancer and specifically, may be a brain tumor such as glioma, glioblastoma, meningioma, pituitary adenoma, and schwannoma.

In an exemplary embodiment, a1 may be an integer of 0 to 2; and b1 may be an integer of 0 to 2.

In an exemplary embodiment, c1 may be an integer of 0 to 2.

In an exemplary embodiment, -̅ -̅ -̅ may be a double bond; Ar¹ may be 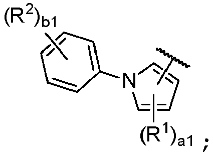 R² may be haloC1-C4alkyloxy or C6-C12arylC1-C4alkyloxy; a1 may be an integer of 0 to 2; b1 may be an integer of 0 to 2; R^{A} may be hydrogen, C1-C4alkyl, C3-C8cycloalkyl, or -L¹-Het¹; L¹ may be C1-C4alkylene; Het¹ may be C3-C8heterocycloalkyl.

In an exemplary embodiment, -̅ -̅ -̅ may be a double bond; Ar¹ may be 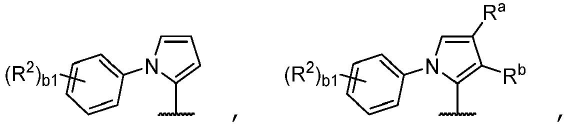 or 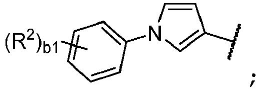 R^{a} and R^{b} may be independently of each other C1-C4alkyl; R² may be haloC1-C4alkyloxy, or C6-C12arylC1-C4alkyloxy; b1 may be an integer of 0 to 2; R^{A} may be hydrogen, C1-C4alkyl, C3-C8cycloalkyl, or 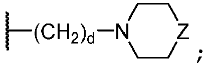 Z may be O or S; and d may be an integer of 1 to 4.

In an exemplary embodiment, -̅ -̅ -̅ may be a single bond or double bond; Ar¹ may be 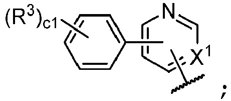 X¹ may be CH or N; R³ may be C1-C4alkyl, haloC1-C4alkyl, C1-C4alkoxy, haloC1-C4alkoxy, or C6-C12arylC1-C4alkyloxy; c1 may be an integer of 0 to 2; R^{A} may be hydrogen, C1-C4alkyl, C3-C8cycloalkyl, or -L¹-Het¹; L¹ may be C1-C4alkylene; and Het¹ may be C3-C8heterocycloalkyl.

In an exemplary embodiment, -̅ -̅ -̅ may be a single bond or double bond; Ar¹ may be 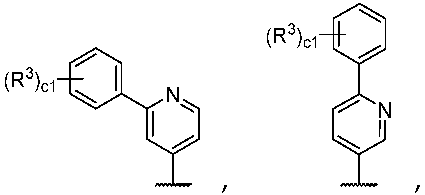 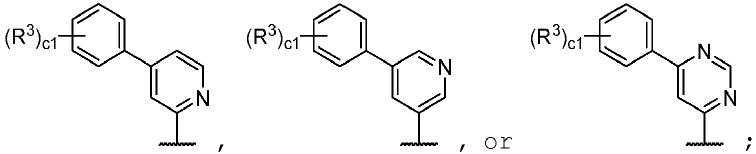 R³ may be C1-C4alkyl, haloC1-C4alkyl, C1-C4alkoxy, haloC1-C4alkoxy, or C6-C12arylC1-C4alkyloxy; c1 may be an integer of 0 to 2; R^{A} may be hydrogen, C1-C4alkyl, C3-C8cycloalkyl, or 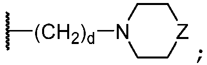 Z may be O or S; and d may be an integer of 1 to 4.

In an exemplary embodiment, Ar¹ may be selected from the following structures:

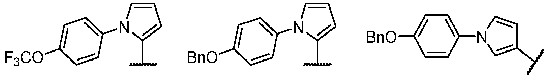

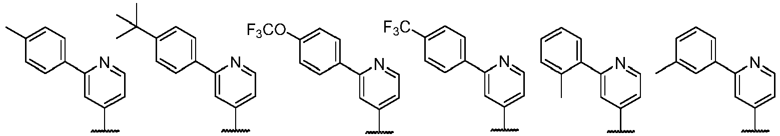

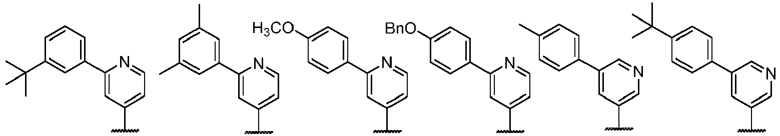

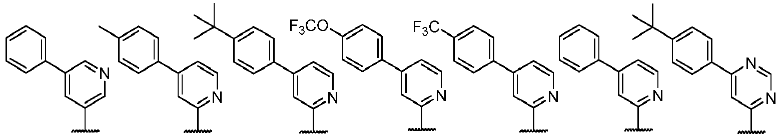

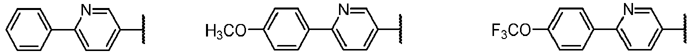

In an exemplary embodiment, R^{A} may be hydrogen, methyl, ethyl, or

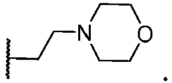

In an exemplary embodiment, the substituted thiazolidinedione derivative compound may be any one selected from the following compound group, but is not limited thereto:
(*Z*)-5-((1-(4-(trifluoromethoxy)phenyl)-1*H*-pyrrol-2-yl)methylene)thiazolidin-2,4-dione;
(*Z*)-5-((1-(4-(benzyloxy)phenyl)-1*H*-pyrrol-2-yl)methylene)thiazolidin-2,4-dione;
(*Z*)-5-((1-(4-(benzyloxy)phenyl)-1*H*-pyrrol-3-yl)methylene)thiazolidin-2,4-dione;
(*Z*)-5-((2-(*p*-tolyl)pyridin-4-yl)methylene)thiazolidin-2,4-dione;
(*Z*)-5-((2-(4-(*t*-butyl)phenyl)pyridin-4-yl)methylene)thiazolidin-2,4-dione;
(*Z*)-5-((2-(4-(trifluoromethyl)phenyl)pyridin-4-yl)methylene)thiazolidin-2,4-dione;
(*Z*)-5-((2-(4-methoxyphenyl)pyridin-4-yl)methylene)thiazolidin-2,4-dione;
(*Z*)-5-((2-(4-(trifluoromethoxy)phenyl)pyridin-4-yl)methylene)thiazolidin-2,4-dione;
(*Z*)-5-((2-(4-(benzyloxy)phenyl)pyridin-4-yl)methylene)thiazolidin-2,4-dione;
(*Z*)-5-((2-(*o*-tolyl)pyridin-4-yl)methylene)thiazolidin-2,4-dione;
(Z)-5-((2-(*m*-tolyl)pyridin-4-yl)methylene)thiazolidin-2,4-dione;
(*Z*)-5-((2-(3-(*t*-butyl)phenyl)pyridin-4-yl)methylene)thiazolidin-2,4-dione;
(*Z*)-5-((2-(3,5-dimethylphenyl)pyridin-4-yl)methylene)thiazolidin-2,4-dione;
5-((2-(4-(*t*-butyl)phenyl)pyridin-4-yl)methyl)thiazolidin-2,4-dione;
(*Z*)-5-((2-(4-(*t*-butyl)phenyl)pyridin-4-yl)methylene)-3-methylthiazolidin-2,4-dione;
(*Z*)-5-((2-(4-(*t*-butyl)phenyl)pyridin-4-yl)methylene)-3-(2-morpholinoethyl)thiazolidin-2,4-dione;
*(Z)-5-(*(6-phenylpyridin-3-yl)methylene)thiazolidin-2,4-dione;
(*Z*)-5-((6-(4-methoxyphenyl)pyridin-3-yl)methylene)thiazolidin-2,4-dione;
(*Z*)-5-((6-(4-(trifluoromethoxy)phenyl)pyridin-3-yl)methylene)thiazolidin-2,4-dione;
(*Z*)-5-((5-phenylpyridin-3-yl)methylene)thiazolidin-2,4-dione;
(*Z*)-5-((5-(*p*-tolyl)pyridin-3-yl)methylene)thiazolidin-2,4-dione;
(*Z*)-5-((5-(4-(*t*-butyl)phenyl)pyridin-3-yl)methylene)thiazolidin-2,4-dione;
(*Z*)-5-((4-phenylpyridin-2-yl)methylene)thiazolidin-2,4-dione;
(*Z*)-5-((4-(*p*-tolyl)pyridin-2-yl)methylene)thiazolidin-2,4-dione;
(*Z*)-5-((4-(4-(*t*-butyl)phenyl)pyridin-2-yl)methylene)thiazolidin-2,4-dione;
(*Z*)-5-((4-(4-(trifluoromethyl)phenyl)pyridin-2-yl)methylene)thiazolidin-2,4-dione;
(*Z*)-5-((4-(4-(trifluoromethoxy)phenyl)pyridin-2-yl)methylene)thiazolidin-2,4-dione; and
(*Z*)*-*5-((6-(4-(*t-*butyl)phenyl)pyrimidin-4-yl)methylene)thiazolidin-2,4-dione.

The compounds described above may be prepared based on the examples described later. In the examples described later, representative examples are described, but if necessary, a substituent may be added or excluded, and the position of the substituent may be changed. In addition, a starting material, a reactant, a reaction condition, and the like may be changed, based on the technology known in the art. Changing of the type or position of substituents of the remaining positions as needed may be performed by a person skilled in the art using the technology known in the art. More details are described in the following examples.

The substituted thiazolidinedione derivative compound according to an exemplary embodiment includes not only a pharmaceutically acceptable salt thereof but also all possible hydrates and solvates which may be prepared therefrom.

Since the substituted thiazolidinedione derivative compound may be used after prepared into the forms of a prodrug, a hydrate, a solvent, and a pharmaceutically acceptable salt in order to increase in vivo adsorption or solubility, the forms of a prodrug, a hydrate, a solvent, or a pharmaceutically acceptable salt as well as the compound of Chemical Formula I belong to the scope of the present invention.

The substituted thiazolidinedione derivative compound according to an exemplary embodiment may be used in the form of a pharmaceutically acceptable salt, and the pharmaceutically acceptable salt is a salt prepared according to a common method in the art, and the preparation method thereof is known to a person skilled in the art. Specifically, the pharmaceutically acceptable salt includes salts derived from free acids and bases which are pharmacologically or physiologically acceptable, but is not limited thereto.

An acid addition salt formed by the pharmaceutically acceptable free acid is obtained from inorganic acids such as hydrochloric acid, nitric acid, phosphoric acid, sulfuric acid, hydrobromic acid, hydroiodic acid, nitrous acid, and phosphorous acid, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, and hydroiodic acid. The kind of pharmaceutically non-toxic salt as such includes sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate , propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, mandelate, and the like.

The acid addition salt may be prepared by a common method, and for example, may be prepared by dissolving the substituted thiazolidinedione derivative compound according to an exemplary embodiment in a water miscible organic solvent such as methanol, ethanol, acetone, dichloromethane, and acetonitrile, adding an organic acid or inorganic acid to produce a precipitate, and filtering and drying the precipitate, or by distilling a solvent and an excessive amount of acid under reduced pressure, performing drying, and performing crystallization under an organic solvent.

In addition, a pharmaceutically acceptable metal salt may be prepared using a base. An alkali metal salt or an alkali earth metal salt is obtained by, for example, dissolving the substituted thiazolidinedione derivative compound according to an exemplary embodiment in an excessive amount of an alkali metal hydroxide or alkali earth metal hydroxide solution, filtering the undissolved compound salt, and then evaporating and drying a filtrate. Herein, it is pharmaceutically appropriate to prepare a sodium, potassium, or calcium salt as a metal salt, but which is not limited thereto. In addition, a silver salt corresponding thereto may be obtained by reacting the alkali metal or alkali earth metal salt with an appropriate silver salt (e.g., silver nitrate).

The hydrate of the substituted thiazolidinedione derivative compound according to an exemplary embodiment refers to the compound or the pharmaceutically acceptable salt thereof including a stoichiometric or nonstoichiometric amount of water bonded by a non-covalent intermolecular force.

The solvate of the substituted thiazolidinedione derivative compound according to an exemplary embodiment refers to the compound or the pharmaceutically acceptable salt thereof which includes a stoichiometric or nonstoichiometric amount of solvent bonded by a non-covalent intermolecular force. An available solvent includes a volatile and non-toxic solvent.

That is, the substituted thiazolidinedione derivative compound according to an exemplary embodiment is dissolved in a water-compatible solvent such as methanol, ethanol, acetone, and 1,4-dioxane, and then a free acid or a free base is added thereto to perform crystallization or recrystallization, thereby forming a solvate including a hydrate.

Another exemplary embodiment provides a pharmaceutical composition for preventing or treating cancer including a substituted thiazolidinedione derivative compound represented by the following Chemical Formula 2, a hydrate thereof, or a pharmaceutically acceptable salt thereof as an effective component:

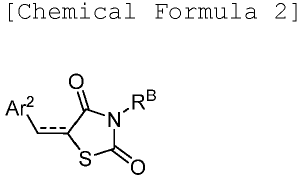

wherein
-̅ -̅ -̅ is a single bond or double bond;
Ar² is 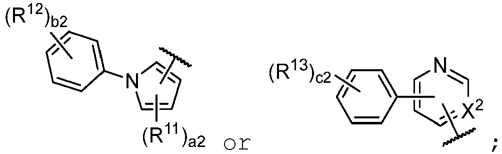
R¹¹ is C1-C10alkyl;
R¹² is C1-C10alkyl, hydroxy, haloC1-C10alkyloxy, or C6-C12arylC1-C10alkyloxy;
R¹³ is C1-C10alkyl, haloC1-C10alkyl, C1-C10alkoxy, haloC1-C10alkoxy, or C6-C12arylalkyloxy;
X² is CH or N;
R^{B} is hydrogen, C1-C10alkyl, C3-C10cycloalkyl, C6-C12aryl, C6-C12arylC1-C10alkyl, or -L²-Het²;
L² is C1-C10alkylene;
Het² is C3-C10heterocycloalkyl;
a2 is an integer of 0 to 3; and
b2 and c2 are independently of each other an integer of 0 to 5.

In an exemplary embodiment, the pharmaceutical composition may be used for the purpose of treating, preventing, or alleviating a cancer disease caused by abnormal cell growth. The cancer disease which may be prevented, treated, or alleviated by the treatment with the pharmaceutical composition may be a brain tumor.

Specifically, the pharmaceutical composition may selectively inhibit sterol regulatory element-binding protein 1 (SREBP1), and by selectively inhibiting SREBP1, the prevention or treatment effect of cancer may be expressed. Thus, the pharmaceutical composition may act as a SREBP1 inhibitor and specifically induce apoptosis in cancer cell lines, in particular, a brain tumor, it may be useful as an agent for efficiently ameliorating, preventing, and treating a brain tumor having an epidermal growth factor (EGF) signal-dependent anticancer agent resistance mechanism without side effects.

According to a specific example, SREBP1 is inhibited by treatment with the substituted thiazolidinedione derivative compound to inhibit brain tumor cell proliferation. Thus, the substituted thiazolidinedione derivative compound according to the present invention selectively inhibits SREBP1 in cancer tissues such as a brain tumor, thereby inhibiting cancer cell proliferation, and thus, may be useful for treating or preventing a cancer disease.

In Chemical Formula 2 according to an exemplary embodiment, a2 may be an integer of 0 to 2; and b2 may be an integer of 0 to 2.

In Chemical Formula 2 according to an exemplary embodiment, c2 may be an integer of 0 to 2.

In Chemical Formula 2 according to an exemplary embodiment, -̅ -̅ -̅ may be a double bond; Ar² may be 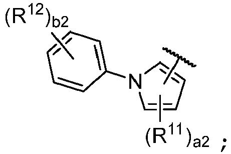 R¹² may be C1-C10alkyl, hydroxy, haloC1-C10alkyloxy, or C6-C12arylC1-C10alkyloxy; a2 may be an integer of 0 to 2; b2 may be an integer of 0 to 2; R^{B} may be hydrogen, C1-C4alkyl, C3-C8cycloalkyl, or -L²-Het²; L² may be C1-C4 alkylene; and Het² may be C3-C8 heterocycloalkyl.

In Chemical Formula 2 according to an exemplary embodiment, -̅ -̅ -̅ may be a double bond; Ar² may be 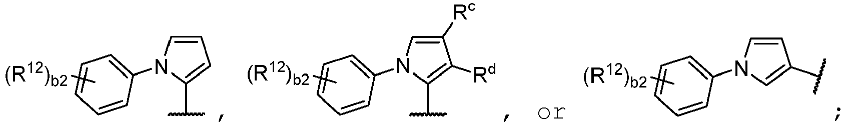 R^{c} and R^{d} may be independently of each other C1-C4alkyl; R¹² may be C1-C4alkyl, hydroxy, haloC1-C4alkyloxy, or C6-C12arylC1-C4alkyloxy; b2 may be an integer of 0 to 2; R^{B} may be hydrogen, C1-C4alkyl, C3-C8cycloalkyl, or 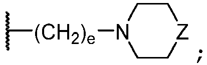 Z may be O or S; and e may be an integer of 1 to 4.

In Chemical Formula 2 according to an exemplary embodiment, -̅ -̅ -̅ may be a single bond or double bond; Ar² may be 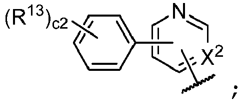 X² may be CH or N; R¹³ may be C1-C4alkyl, haloC1-C4alkyl, C1-C4alkoxy, haloC1-C4alkoxy, or C6-C12arylC1-C4alkyloxy; c2 may be an integer of 0 to 2; R^{B} may be hydrogen, C1-C4alkyl, C3-C8cycloalkyl, or -L²-Het²; L² may be C1-C4alkylene; and Het² may be C3-C8heterocycloalkyl.

In Chemical Formula 2 according to an exemplary embodiment, -̅ -̅ -̅ may be a single bond or double bond; Ar² may be 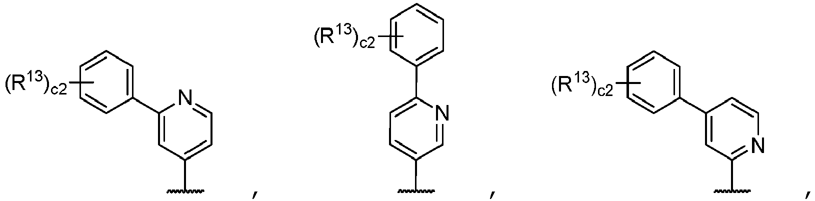 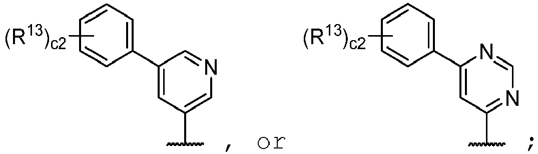 R¹³ may be C1-C4alkyl, haloC1-C4alkyl, C1-C4alkoxy, haloC1-C4alkoxy, or C6-C12arylC1-C4alkyloxy; c2 may be an integer of 0 to 2; R^{B} may be hydrogen, C1-C4alkyl, C3-C8cycloalkyl, or 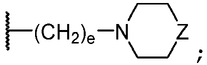 Z may be O or S; and e may be an integer of 1 to 4.

In Chemical Formula 2 according to an exemplary embodiment, Ar² may be selected from the following structures:

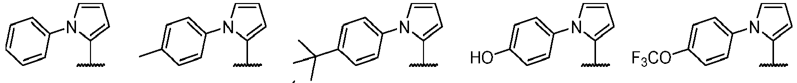

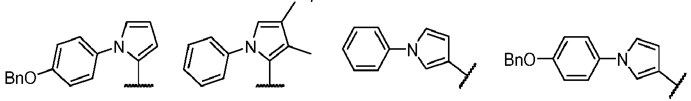

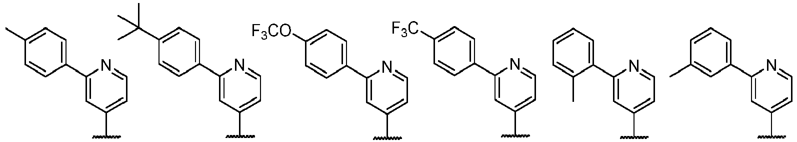

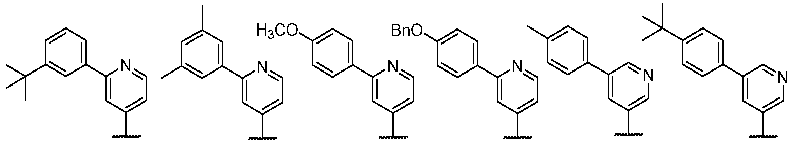

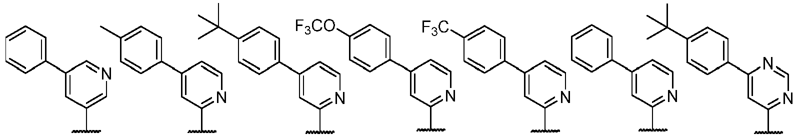

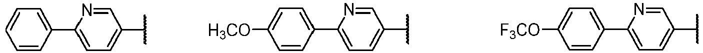

In Chemical Formula 2 according to an exemplary embodiment, R^{B} may be hydrogen, methyl, ethyl, or

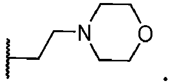

The substituted thiazolidinedione derivative compound of Chemical Formula 2 according to an exemplary embodiment may be any one selected from the following compound group, but is not limited thereto:
(*Z*)-5-((1-phenyl-1*H*-pyrrol-2-yl)methylene)thiazolidin-2,4-dione;
(*Z*)-5-((1-(*p*-tolyl)-1H-pyrrol-2-yl)methylene)thiazolidin-2,4-dione;
(*Z*)-5-((1-(4-(*t*-butyl)phenyl)-1*H*-pyrrol-2-yl)methylene)thiazolidin-2,4-dione;
(*Z*)-5-((1-(4-hydroxyphenyl)-1*H*-pyrrol-2-yl)methylene)thiazolidin-2,4-dione;
(*Z*)-5-((1-(4-(trifluoromethoxy)phenyl)-1*H*-pyrrol-2-yl)methylene)thiazolidin-2,4-dione;
(*Z*)-5-((1-(4-(benzyloxy)phenyl)-1*H*-pyrrol-2-yl)methylene)thiazolidin-2,4-dione;
(*Z*)-5-((3,4-dimethyl-1-phenyl-1*H*-pyrrol-2-yl)methylene)thiazolidin-2,4-dione;
(*Z*)-5-((1-phenyl-1*H*-pyrrol-3-yl)methylene)thiazolidin-2,4-dione;
(*Z*)-5-((1-(4-(benzyloxy)phenyl)-1*H*-pyrrol-3-yl)methylene)thiazolidin-2,4-dione;
(*Z*)-5-((2-(*p*-tolyl)pyridin-4-yl)methylene)thiazolidin-2,4-dione;
(*Z*)-5-((2-(4-(*t*-butyl)phenyl)pyridin-4-yl)methylene)thiazolidin-2,4-dione;
(*Z*)-5-((2-(4-(trifluoromethyl)phenyl)pyridin-4-yl)methylene)thiazolidin-2,4-dione;
(*Z*)-5-((2-(4-methoxyphenyl)pyridin-4-yl)methylene)thiazolidin-2,4-dione;
(*Z*)-5-((2-(4-(trifluoromethoxy)phenyl)pyridin-4-yl)methylene)thiazolidin-2,4-dione;
(*Z*)-5-((2-(4-(benzyloxy)phenyl)pyridin-4-yl)methylene)thiazolidin-2,4-dione;
(*Z*)-5-((2-(*o*-tolyl)pyridin-4-yl)methylene)thiazolidin-2,4-dione;
(*Z*)-5-((2-(*m*-tolyl)pyridin-4-yl)methylene)thiazolidin-2,4-dione;
(*Z*)-5-((2-(3-(*t*-butyl)phenyl)pyridin-4-yl)methylene)thiazolidin-2,4-dione;
(*Z*)-5-((2-(3,5-dimethylphenyl)pyridin-4-yl)methylene)thiazolidin-2,4-dione;
5-((2-(4-(*t*-butyl)phenyl)pyridin-4-yl)methyl)thiazolidin-2,4-dione;
(*Z*)-5-((2-(4-(*t*-butyl)phenyl)pyridin-4-yl)methylene)-3-methylthiazolidin-2,4-dione;
(*Z*)-5-((2-(4-(t-butyl)phenyl)pyridin-4-yl)methylene)-3-(2-morpholinoethyl)thiazolidin-2,4-dione;
(*Z*)-5-((6-phenylpyridin-3-yl)methylene)thiazolidin-2,4-dione;
(*Z*)-5-((6-(4-methoxyphenyl)pyridin-3-yl)methylene)thiazolidin-2,4-dione;
(Z)-5-((6-(4-(trifluoromethoxy)phenyl)pyridin-3-yl)methylene)thiazolidin-2,4-dione;
(*Z*)-5-((5-phenylpyridin-3-yl)methylene)thiazolidin-2,4-dione;
(*Z*)-5-((5-(*p*-tolyl)pyridin-3-yl)methylene)thiazolidin-2,4-dione;
(*Z*)-5-((5-(4-(*t*-butyl)phenyl)pyridin-3-yl)methylene)thiazolidin-2,4-dione;
(*Z*)-5-((4-phenylpyridin-2-yl)methylene)thiazolidin-2,4-dione;
(*Z*)-5-((4-(*p*-tolyl)pyridin-2-yl)methylene)thiazolidin-2,4-dione;
(*Z*)-5-((4-(4-(*t*-butyl)phenyl)pyridin-2-yl)methylene)thiazolidin-2,4-dione;
(*Z*)-5-((4-(4-(trifluoromethyl)phenyl)pyridin-2-yl)methylene)thiazolidin-2,4-dione;
(*Z*)-5-((4-(4-(trifluoromethoxy)phenyl)pyridin-2-yl)methylene)thiazolidin-2,4-dione; and
(*Z*)-5-((6-(4-(*t*-butyl)phenyl)pyrimidin-4-yl)methylene)thiazolidin-2,4-dione.

The pharmaceutical composition according to an exemplary embodiment further includes a common non-toxic pharmaceutically acceptable carrier and/or excipient in addition to the effective component and may be formulated into a common preparation in the pharmaceutical field, that is, a preparation for oral administration or a preparation for parenteral administration. In addition, a diluent such as a filler, an extender, a binder, a wetting agent, a disintegrant, and a surfactant may be further included.

The pharmaceutically acceptable carrier, excipient, or diluent may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, dextrin, cyclodextrin, for example, α-, β-, and γ-cyclodextrin, hydroxyalkyl cyclodextrin (including 2-and 3-hydroxypropyl-β-cyclodextrin), talc, magnesium stearate, mineral oil, or the like, but is not limited thereto.

The pharmaceutical composition may be formulated into various forms, for example, preparations for oral administration such as powders, granules, tablets, capsules, suspensions, emulsions, syrups, and aerosols, injections of sterile injection solutions, and the like by a common method according to the purpose of use, and may be orally administered or administered by various routes including intravenous, intraperitoneal, subcutaneous, rectal, and topical administrations.

The preparation for oral administration may be selectively enteric coated, and show delayed or sustained release by enteric coating. That is, the preparation for oral administration may be a formation having an immediate or modified release pattern.

The preparation for parenteral administration may be a formulation having an immediate or modified release pattern, and the modified release pattern may be a delayed or sustained release pattern.

The pharmaceutical composition according to an exemplary embodiment may further include a filler, an anticoagulant, a lubricant, a wetting agent, a flavoring, an emulsifying agent, an antiseptic agent, and the like.

An example of a formulation for oral administration may include tablets, pills, hard/soft capsules, solutions, suspensions, emulsifiers, syrups, granules, elixirs, and the like, and these formulations may use one or more of commonly used diluents or excipients such as fillers, extenders, wetting agents, disintegrants, lubricants, binders, and surfactants, in addition to the effective component. As a disintegrant, agar, starch, alginic acid or a sodium salt thereof, an anhydrous calcium monohydrogen phosphate salt, and the like may be used, as a lubricant, silica, talc, stearic acid, or a magnesium salt or calcium salt thereof, polyethylene glycol, and the like may be used, and as a binder, magnesium aluminum silicate, starch paste, gelatin, tragacanth, methyl cellulose, sodium carboxymethyl cellulose, polyvinylpyrrolidone, low-substituted hydroxypropyl cellulose, and the like may be used. Other than that, lactose, dextrose, sucrose, mannitol, sorbitol, cellulose, glycine, and the like may be used as a lubricant, and if necessary, commonly known effervescent mixtures, absorbents, colorants, flavoring agents, sweetening agents, and the like may be used therewith.

An example of a preparation for parenteral administration may include sterile aqueous solutions, nonaqueous solvent, suspensions, emulsions, freeze-dried preparations, suppositories, and the like. As the nonaqueous solvent and the suspension, propylene glycol, polyethylene glycol, a vegetable oil such as an olive oil, an injectable ester such as ethyl oleate, and the like may be used. As a base of the suppository, witepsol, macrogol, tween 61, cacao butter, laurin butter, glycerol, gelatin, and the like may be used. Meanwhile, an injection may include a conventional additive such as a solubilizer, an isotonic agent, a suspending agent, an emulsifying agent, a stabilizer, and an antiseptic agent. For formulation into the injection, an effective component is mixed in water with a stabilizer or a buffer to prepare a solution or a suspension, which may be produced into a unit dosage form of an ampule or vial.

The pharmaceutical composition according to an exemplary embodiment may be sterilized, may further include an adjuvant such as an antiseptic agent, a stabilizer, a thickener, a hydrating agent or an emulsifying accelerator, a salt for regulating osmotic pressure, and/or a buffer, or may further include other therapeutically useful materials, and may be formulated according to a conventional method such as dissolution, dispersion, mixing, granulation, gelling, or coating.

The pharmaceutically effective amount of the substituted thiazolidinedione derivative compound which is an effective component in the pharmaceutical composition according to an exemplary embodiment may be determined by factors including the health state of a patient, the kind of diseases, severity, an activity of a drug, a sensitivity to a drug, an administration manner, an administration time, administration route and releasing rate, a treatment period, combination, and a simultaneously used drug, and other factors well known in the medical field. Specifically, the effective amount of the substituted thiazolidinedione derivative compound in the pharmaceutical composition may vary depending on the age, the gender, and the weight of a patient, and generally, about 0.001 to 500 mg/kg/day, preferably 0.01 to 100 mg/kg/day may be administered every day or every other day or administered in a divided dose of 1 to several times a day. However, since the amount may be increased or decreased depending on an administration route, severity of the disease, gender, weight, age, and the like, the administration amount does not limit the scope of the present invention in any way.

The pharmaceutical composition according to an exemplary embodiment may be orally or parenterally administered, and the substituted thiazolidinedione derivative compound may enter a gastrointestinal tract by oral administration as an anticancer agent for oral administration in order to treat cancer, or, for example, may be directly absorbed through bloodstream from the mouth, like buccal or sublingual administration.

The pharmaceutical composition according to an exemplary embodiment may be administered as an individual therapeutic agent or in combination with other therapeutic agents, may be sequentially or simultaneously administered with a conventional therapeutic agent, and may be administered in a single or multiple doses. Taking the factors into account, it is important to administer the composition in a minimum amount to obtain a maximum effect without any side effect, and this will be easily determined by a person skilled in the art.

Another exemplary embodiment provides a health functional food composition for preventing or ameliorating cancer including the substituted thiazolidinedione derivative compound, the hydrate thereof, or the sitologically acceptable salt thereof.

The sitologically acceptable salt may be obtained by reacting the compound of the present invention with an inorganic acid such as hydrochloric acid, bromic acid, sulfuric acid, nitric acid, and phosphoric acid, a sulfonic acid such as methanesulfonic acid, ethanesulfonic acid, and p-toluenesulfonic acid, or an organic carbonic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. In addition, it may be obtained by reacting the compound of the present invention with a base to form an ammonium salt, an alkali metal salt such as sodium or potassium salt, alkaline earth metal salt such as calcium or magnesium salt, a salt of organic bases such as dicyclohexylamine, N-methyl-D-glucamine, and tris(hydroxymethyl)methylamine, and an amino acid salt such as arginine and lysine, and is not limited thereto.

The health functional food composition may be provided in the form of powder, granules, tablets, capsules, a syrup, or a beverage, and the health functional food is used with other food or a food additive in addition to the compound of the present invention as the effective component and may be appropriately used according to a common method. The mixed amount of the effective component may be appropriately determined according to its purpose of use, for example, prevention, health, or therapeutic treatment.

The health functional food composition may include various nutritional supplements, vitamins, minerals (electrolyte), flavors such as synthetic flavors and natural flavors, colorants and enhancers (cheese, chocolate, etc.), pectic acid and salts thereof, alginic acid and salts thereof, organic acids, protective colloid thickeners, pH regulators, stabilizers, preservatives, glycerin, alcohol, carbonation agent used in carbonated drink, and the like. Other than that, fruit pulp for preparing natural fruit juice, fruit juice drink, and vegetable drink may be included. These components may be used independently or in combination.

In addition, the health functional food may further include food additives, and whether it is appropriate as "food additives" is determined by the specification and the standards for the relevant item in accordance with the general rules and general test methods of the food additive code approved by the Korea Food & Drug Administration, unless otherwise stipulated.

The items listed in the "food additive code" may include, for example, chemical synthetics such as ketones, glycine, potassium citrate, nicotinic acid, and cinnamic acid, natural additives such as persimmon pigment, licorice extract, crystalline cellulose, and guar gum, and mixed preparations such as sodium L-glutamate preparation, alkaline additives for noodles, preservative preparation, and tar colorant preparation.

The substituted thiazolidinedione derivative compound of the present invention included in the health functional food composition may be used according to the effective dose of the pharmaceutical composition, but in the case of longterm intake for the purpose of health and hygiene or for the purpose of health control, may be used below the range, and since the effective component has no problem in terms of safety, it may be used in the amount above the range, of course.

The health functional food composition may be formulated into various formulations such as meat, sausage, bread, chocolate, candies, snacks, confectionery, pizza, ramen, other noodles, chewing gum, dairy products including ice cream, various soups, beverages, tea, drinks, alcoholic beverages, and vitamin complexes.

Another exemplary embodiment provides a selective inhibitor of SREBP1 including the substituted thiazolidinedione derivative compound, the hydrate thereof, or the pharmaceutically acceptable salt thereof as an effective component.

Another exemplary embodiment provides a method for preventing or treating cancer including administering the substituted thiazolidinedione derivative compound, the hydrate thereof, the pharmaceutically acceptable salt thereof, or the pharmaceutical composition to an individual who developed cancer or is at risk of developing cancer.

In addition, the present invention provides the substituted thiazolidinedione derivative compound, the hydrate thereof, or the pharmaceutically acceptable salt thereof for use in treatment of cancer.

In addition, the present invention provides a use of the substituted thiazolidinedione derivative compound, the hydrate thereof, or the pharmaceutically acceptable salt thereof for use in preparation of a pharmaceutical agent for treating cancer.

Hereinafter, the examples and the experimental examples will be illustrated specifically in detail in the following. However, the examples and the experimental examples described later are only illustrative of some, and the technology described in the present specification is not construed as being limited thereto.

### Example I: Synthesis of phenyl-pyrrole skeleton compound

### [Example 1] Preparation of (Z)-5-((1-phenyl-1H-pyrrol-2-yl)methylene)thiazolidine-2,4-dione (Compound 1)

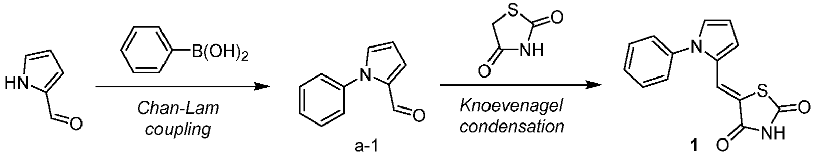

### Step 1: Preparation of 1-phenyl-1H-pyrrole-2-carbaldehyde (Compound a-1)

1*H*-Pyrrole-2-carbaldehyde (200 mg, 2.1 mmol), copper(II) acetate hydrate (840 mg, 4.2 mmol), and triethylamine (851 mg, 8.4 mmol) were added to dichloromethane (15 ml), phenylboronic acid (769 mg, 6.31 mmol) was added dropwise, and then stirring was performed at room temperature for 48 hours. After completing the reaction, an organic layer obtained by extraction using EtOAc and water was washed with brine, dried with magnesium sulfate, and filtered. A residue obtained by distilling the filtered solution under reduced pressure was purified by medium pressure liquid chromatography to obtain 1-phenyl-1*H-*pyrrole-2-carbaldehyde (Compound a-1) (158 mg, yield: 44%).

¹H NMR (400 MHz, CDCl₃) δ 9.55 (s, 1H), 7.50 - 7.37 (m, 3H), 7.37 - 7.31 (m, 2H), 7.16 (dd, *J* = 4.0, 1.7 Hz, 1H), 7.06 (t, *J* = 1.9 Hz, 1H), 6.39 (dd, *J* = 4.0, 2.6 Hz, 1H).

### Step 2: Preparation of (Z)-5-((1-phenyl-1H-pyrrol-2-yl)methylene)thiazolidine-2,4-dione (Compound 1)

Compound a-1 (50 mg, 0.292 mmol) obtained in Step 1, thiazolidine-2,4-dione (37 mg, 0.321 mmol), piperidine (0.029 ml, 0.292 mmol), and acetic acid (0.017 ml, 0.292 mmol) were dissolved in toluene (5 ml), and then stirring was performed at 100°C for 2 hours. After completing the reaction, an organic layer obtained by extraction using EtOAc and water was washed with brine, dried with magnesium sulfate, and filtered. A residue obtained by distilling the filtered solution under reduced pressure was purified using medium pressure liquid chromatography (silica gel, 0-30% EtOAc/hexane) to obtain a compound. The obtained compound was recrystallized using EtOAc to obtain a solidified final target compound, (Z)-5-((1-phenyl-1*H*-pyrrol-2-yl)methylene)thiazolidine-2,4-dione (Compound 1) (28 mg, yield: 35%).

¹H NMR (400 MHz, DMSO-*d₆*) δ 12.37 (br s, 1H), 7.65 - 7.52 (m, 3H), 7.46 - 7.41 (m, 3H), 7.30 (s, 1H), 6.72 (d, *J* = 3.8 Hz, 1H), 6.57 - 6.53 (m, 1H); LCMS (ESI), m/z = 270.95 [M+H]⁺.

### [Example 2] Preparation of (Z)-5-((1-(p-tolyl)-1H-pyrrol-2-yl)methylene)thiazolidine-2,4-dione (Compound 2)

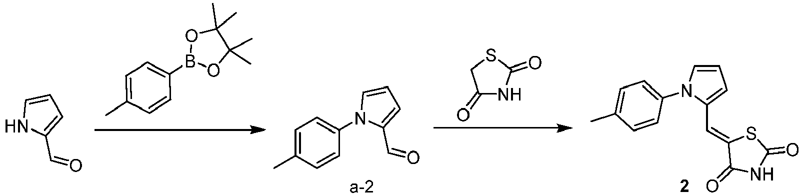

### Step 1: Preparation of 1-(p-tolyl)-1H-pyrrole-2-carbaldehyde (Compound a-2)

1-(*p*-Tolyl)-1*H*-pyrrole-2-carbaldehyde (Compound a-2) was obtained (180 mg, yield: 30%) in the same manner as in Step 1 of Example 1, except that 4,4,5,5-tetramethyl-2-(*p-*tolyl)-1,3,2-dioxaborolane was used instead of phenylboronic acid.

¹H NMR (400 MHz, CDCl₃) δ 9.55 (s, 1H), 7.27 - 7.20 (m, 4H), 7.15 (dd, *J* = 4.0, 1.6 Hz, 1H), 7.05 - 7.02 (m, 1H), 6.38 (dd, *J* = 3.9, 2.6 Hz, 1H), 2.41 (s, 3H).

### Step 2: Preparation of (Z)-5-((1-(p-tolyl)-1H-pyrrol-2-yl)methylene)thiazolidine-2,4-dione (Compound 2)

The target compound, (*Z*)-5-((1-(*p*-tolyl)-1*H*-pyrrol-2-yl)methylene)thiazolidine-2,4-dione (Compound 2), was obtained (25 mg, yield: 53%) in the same manner as in Step 2 of Example 1, except that Compound a-2 (0.162 mmol) was used instead of Compound a-1.

¹H NMR (400 MHz, (CD₃)₂CO) δ 10.88 (br s, 1H), 7.44 (s, 1H), 7.41 (d, *J* = 8.0 Hz, 2H), 7.34 - 7.29 (m, 2H), 7.27 (dd, *J* = 2.7, 1.4 Hz, 1H), 6.74 (dd, *J* = 4.1, 0.9 Hz, 1H), 6.56 - 6.49 (m, 1H), 2.44 (s, 3H); LCMS (ESI), m/z = 284.93 [M+H]⁺.

### [Example 3] Preparation of (Z)-5-((1-(4-(tert-butyl)phenyl)-1H-pyrrol-2-yl)methylene)thiazolidine-2,4-dione (Compound 3)

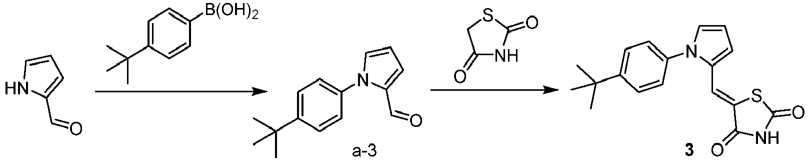

### Step 1: Preparation of 1-(4-(tert-butyl)phenyl)-1H-pyrrole-2-carbaldehyde (Compound a-3)

1*H*-Pyrrole-2-carbaldehyde (300 mg, 3.15 mmol), copper(II) acetate hydrate (1.26 g, 6.3 mmol), and triethylamine (1.59 g, 15.7 mmol) were added to dichloroethane (15 ml), (4-(*tert*-butyl)phenyl)boronic acid (1.12 g, 3.15 mmol) was added dropwise, and stirring was performed at 80°C for 48 hours. After completing the reaction, an organic layer obtained by extraction using EtOAc and water was washed with brine, dried with magnesium sulfate, and filtered. A residue obtained by distilling the filtered solution under reduced pressure was purified by medium pressure liquid chromatography to obtain 1-(4-(*tert-*butyl)phenyl)-1*H*-pyrrole-2-carbaldehyde (Compound a-3) (130 mg, yield: 18%).

¹H NMR (400 MHz, CDCl₃) δ 9.57 (s, 1H), 7.48 (d, *J* = 8.5 Hz, 2H), 7.29 (d, *J* = 8.5 Hz, 2H), 7.17 (dd, *J* = 4.0, 1.6 Hz, 1H), 7.07 (t, *J* = 1.9 Hz, 1H), 6.40 (dd, *J* = 3.9, 2.7 Hz, 1H), 1.37 (s, 9H).

### Step 2 : Preparation of (Z)-5-((1-(4-(tert-butyl)phenyl)-1H-pyrrol-2-yl)methylene)thiazolidine-2,4-dione (Compound 3)

The reaction was performed with stirring at 100°C for 3 hours in the same manner as in Step 2 of Example 1, except that Compound a-3 (30 mg, 0.132 mmol) was used instead of Compound a-1. After completing the reaction, an organic layer obtained by extraction using EtOAc and water was washed with brine, dried with magnesium sulfate, and filtered. A residue obtained by distilling the filtered solution under reduced pressure was separated by medium pressure liquid chromatography (silica gel, 0-30% EtOAc/Hexane), and further purified using preparative liquid chromatography (30-50% water/MeCN with 0.1% TFA) to obtain a target compound, (Z)-5-((1-(4-(*tert*-butyl)phenyl)-1*H*-pyrrol-2-yl)methylene)thiazolidine-2,4-dione (Compound 3) (20 mg, yield: 46%, TFA salt).

¹H NMR (400 MHz, MeOD) δ 7.61 (d, *J* = 8.5 Hz, 2H), 7.47 (s, 1H), 7.29 (d, *J* = 8.5 Hz, 2H), 7.21 (dd, *J* = 2.3, 1.5 Hz, 1H), 6.75 (d, *J* = 3.4 Hz, 1H), 6.53 - 6.47 (m, 1H), 1.40 (s, 9H); LCMS (ESI), m/z = 326.97 [M+H]⁺.

### [Example 4] Preparation of (Z)-5-((1-(4-hydroxyphenyl)-1H-pyrrol-2-yl)methylene)thiazolidine-2,4-dione (Compound 4)

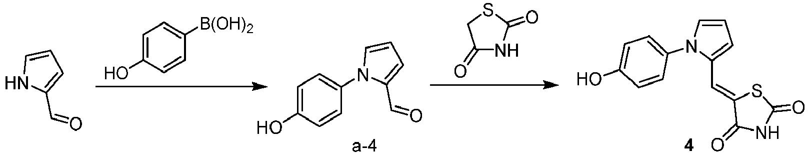

### Step 1: Preparation of 1-(4-hydroxyphenyl)-1H-pyrrole-2-carbaldehyde (Compound a-4)

1-(4-Hydroxyphenyl)-1*H*-pyrrole-2-carbaldehyde (Compound a-4) was obtained (110 mg, yield: 18%) in the same manner as in Step 1 of Example 1, except that (4-hydroxyphenyl)boronic acid was used instead of Phenylboronic acid.

### Step 2: Preparation of (Z)-5-((1-(4-hydroxyphenyl)-1H-pyrrol-2-yl)methylene)thiazolidine-2,4-dione (Compound 4)

The target compound, (*Z*)-5-((1-(4-hydroxyphenyl)-1*H-*pyrrol-2-yl)methylene)thiazolidine-2,4-dione (Compound 4), was obtained (7 mg, yield: 15%) in the same manner as in Step 2 of Example 1, except that Compound a-4 (30 mg, 0.160 mmol) was used instead of Compound a-1.

¹H NMR (400 MHz, MeOD) δ 7.42 (s, 1H), 7.21 - 7.13 (m, 3H), 6.93 (d, *J* = 8.7 Hz, 2H), 6.71 (d, *J* = 3.9 Hz, 1H), 6.49 - 6.45 (m, 1H); LCMS (ESI), m/z = 286.91 [M+H]⁺.

### [Example 5] Preparation of (Z)-5-((1-(4-(trifluoromethoxy)phenyl)-1H-pyrrol-2-yl)methylene)thiazolidine-2,4-dione (Compound 5)

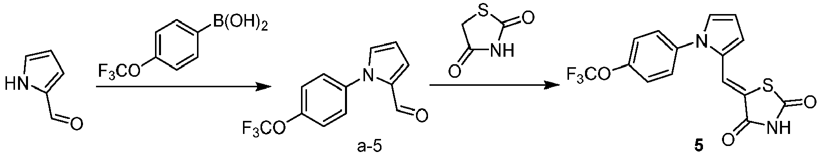

### Step 1: Preparation of 1-(4-(trifluoromethoxy)phenyl)-1H-pyrrole-2-carbaldehyde (Compound a-5)

1-(4-(Trifluoromethoxy)phenyl)-1*H*-pyrrole-2-carbaldehyde (Compound a-5) was obtained (130 mg, yield: 16%) in the same manner as in Step 1 of Example 1, except that (4-(trifluoromethoxy)phenyl)boronic acid was used instead of phenylboronic acid.

### Step 2: Preparation of (Z)-5-((1-(4-(trifluoromethoxy)phenyl)-1H-pyrrol-2-yl)methylene)thiazolidine-2,4-dione (Compound 5)

The target compound, (*Z*)-5-((1-(4-(Trifluoromethoxy)phenyl)-1*H*-pyrrol-2-yl)methylene)thiazolidine-2,4-dione (Compound 5), was obtained (15 mg, yield: 27%) in the same manner as in Step 2 of Example 1, except that Compound a-5 (40 mg, 0.157 mmol) was used instead of Compound a-1.

¹H NMR (400 MHz, MeOD) δ 7.55 - 7.46 (m, 4H), 7.42 (s, 1H), 7.27 (dd, *J* = 2.4, 1.3 Hz, 1H), 6.78 (d, *J* = 3.9 Hz, 1H), 6.57 - 6.51 (m, 1H); LCMS (ESI), m/z = 354.87 [M+H]⁺.

### [Example 6] Preparation of (Z)-5-((1-(4-(benzyloxy)phenyl)-1H-pyrrol-2-yl)methylene)thiazolidine-2,4-dione (Compound 6)

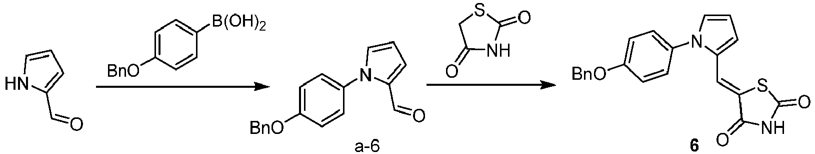

### Step 1: Preparation of 1-(4-(benzyloxy)phenyl)-1H-pyrrole-2-carbaldehyde (Compound a-6)

1-(4-(Benzyloxy)phenyl)-1*H*-pyrrole-2-carbaldehyde (Compound a-6) was obtained (160 mg, yield: 18%) in the same manner as in Step 1 of Example 1, except that (4-(benzyloxy)phenyl)boronic acid was used instead of phenylboronic acid.

¹H NMR (400 MHz, CDCl₃) δ 9.54 (s, 1H), 7.49 - 7.38 (m, 4H), 7.36 (d, *J* = 7.0 Hz, 1H), 7.30 - 7.26 (m, 2H), 7.14 (dd, *J* = 4.0, 1.6 Hz, 1H), 7.04 (dd, *J* = 9.4, 2.6 Hz, 3H), 6.38 (dd, *J* = 3.9, 2.6 Hz, 1H), 5.11 (s, 2H).

### Step 2: Preparation of (Z)-5-((1-(4-(benzyloxy)phenyl)-1H-pyrrol-2-yl)methylene)thiazolidine-2,4-dione (Compound 6)

The target compound, (*Z*)-5-((1-(4-(benzyloxy)phenyl)-1*H*-pyrrol-2-yl)methylene)thiazolidine-2,4-dione (Compound 6), was obtained (28 mg, yield: 41%) in the same manner as in Step 2 of Example 1, except that Compound a-6 (50 mg, 0.180 mmol) was used instead of Compound a-1.

¹H NMR (400 MHz, CDCl₃) δ 7.51 (s, 1H), 7.50 - 7.32 (m, 5H), 7.22 (d, *J* = 8.8 Hz, 2H), 7.13 - 7.03 (m, 3H), 6.74 (d, *J* = 3.8 Hz, 1H), 6.54 - 6.46 (m, 1H), 5.13 (s, 2H); LCMS (ESI), m/z = 377.02 [M+H]⁺.

### [Example 7] Preparation of (Z)-5-((3,4-dimethyl-1-phenyl-1H-pyrrol-2-yl)methylene)thiazolidine-2,4-dione (Compound 7)

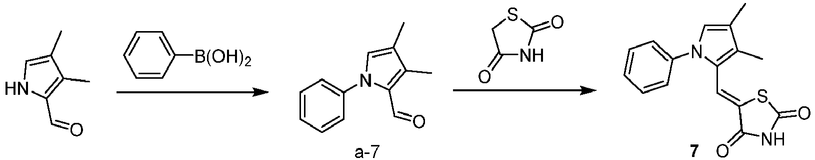

### Step 1: Preparation of 3,4-dimethyl-1-phenyl-1H-pyrrole-2-carbaldehyde (Compound a-7)

3,4-Dimethyl-1-phenyl-1*H*-pyrrole-2-carbaldehyde (Compound a-7) was obtained (210 mg, yield: 65%) in the same manner as in Step 1 of Example 1, except that 3,4-dimethyl-1*H*-pyrrole-2-carbaldehyde was used instead of 1*H*-pyrrole-2-carbaldehyde.

¹H NMR (400 MHz, CDCl₃) δ 9.62 (s, 1H), 7.48 - 7.34 (m, 3H), 7.34 - 7.27 (m, 2H), 6.80 (s, 1H), 2.38 (s, 3H), 2.06 (s, 3H).

### Step 2: Preparation of (Z)-5-((3,4-dimethyl-1-phenyl-1H-pyrrol-2-yl)methylene)thiazolidine-2,4-dione (Compound 7)

The target compound, (*Z*)-5-((3,4-dimethyl-1-phenyl-1*H-*pyrrol-2-yl)methylene)thiazolidine-2,4-dione (Compound 7), was obtained (10 mg, yield: 17%) in the same manner as in Step 2 of Example 1, except that Compound a-7 (40 mg, 0.201 mmol) was used instead of Compound a-1.

¹H NMR (400 MHz, CDCl₃) δ 8.18 (s, 1H), 7.73 (s, 1H), 7.45 - 7.38 (m, 2H), 7.37 - 7.31 (m, 1H), 7.22 (d, *J* = 7.7 Hz, 2H), 6.88 (s, 1H), 2.17 (s, 3H), 2.06 (s, 3H); LCMS (ESI), m/z = 299.03 [M+H]⁺.

### [Example 8] Preparation of (Z)-5-((1-phenyl-1H-pyrrol-3-yl)methylene)thiazolidine-2,4-dione (Compound 8)

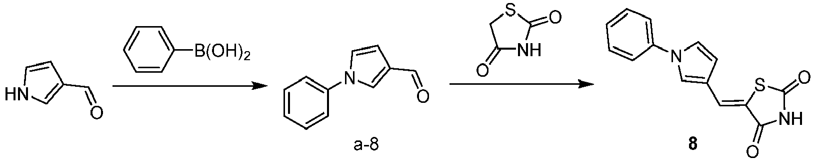

### Step 1: Preparation of 1-phenyl-1H-pyrrole-3-carbaldehyde (Compound a-8)

1-Phenyl-1*H*-pyrrole-3-carbaldehyde (Compound a-8) was obtained (158 mg, yield: 44%) in the same manner as in Step 1 of Example 1, except that 1*H*-pyrrole-3-carbaldehyde was used instead of 1*H*-pyrrole-2-carbaldehyde.

¹H NMR (400 MHz, CDCl₃) δ 9.86 (s, 1H), 7.67 (t, *J* = 1.9 Hz, 1H), 7.52 - 7.45 (m, 2H), 7.42 (dd, *J* = 8.6, 1.3 Hz, 2H), 7.36 (ddd, *J* = 8.3, 2.4, 1.2 Hz, 1H), 7.09 (dd, *J* = 3.7, 1.5 Hz, 1H), 6.81 (dd, J = 2.9, 1.6 Hz, 1H).

### Step 2: Preparation of (Z)-5-((1-phenyl-1H-pyrrol-3-yl)methylene)thiazolidine-2,4-dione (Compound 8)

The target compound, (*Z*)-5-((1-phenyl-1*H*-pyrrol-3-yl)methylene)thiazolidine-2,4-dione (Compound 8), was obtained (25 mg, yield: 52%) in the same manner as in Step 2 of Example 1, except that Compound a-8 (30 mg, 0.175 mmol) was used instead of Compound a-1.

¹H NMR (400 MHz, DMSO-*d₆*) δ 12.31 (s, 1H), 7.93 (t, J = 1.8 Hz, 1H), 7.74 (s, 1H), 7.65 (d, *J* = 7.6 Hz, 2H), 7.61 - 7.59 (m, 1H), 7.52 (t, *J* = 8.0 Hz, 2H), 7.35 (t, *J* = 7.4 Hz, 1H), 6.55 (dd, *J* = 2.9, 1.7 Hz, 1H); LCMS (ESI), m/z = 271.00 [M+H]⁺.

### [Example 9] Preparation of (Z)-5-((1-(4-(benzyloxy)phenyl)-1H-pyrrol-3-yl)methylene)thiazolidine-2,4-dione (Compound 9)

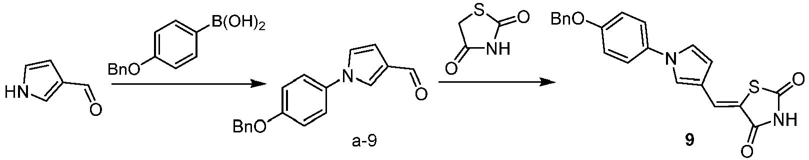

### Step 1: Preparation of 1-(4-(benzyloxy)phenyl)-1H-pyrrole-3-carbaldehyde (Compound a-9)

1-(4-(Benzyloxy)phenyl)-1*H*-pyrrole-3-carbaldehyde (Compound a-9) was obtained (20 mg, yield: 3%) in the same manner as in Step 1 of Example 1, except that 1*H*-pyrrole-3-carbaldehyde and (4-(benzyloxy)phenyl)boronic acid were used instead of 1H-Pyrrole-2-carbaldehyde and phenylboronic acid.

¹H NMR (400 MHz, CDCl₃) δ 9.83 (s, 1H), 7.57 (t, *J* = 1.9 Hz, 1H), 7.47 - 7.29 (m, 7H), 7.07 - 7.03 (m, 2H), 6.99 (t, *J* = 2.3 Hz, 1H), 6.77 (dt, *J* = 5.4, 2.2 Hz, 1H), 5.11 (s, 2H).

### Step 2: Preparation of (Z)-5-((1-(4-(benzyloxy)phenyl)-1H-pyrrol-3-yl)methylene)thiazolidine-2,4-dione (Compound 9)

The target compound, (*Z*)-5-((1-(4-(benzyloxy)phenyl)-1*H*-pyrrol-3-yl)methylene)thiazolidine-2,4-dione (Compound 9), was obtained (33 mg, yield: 77%) in the same manner as in Step 2 of Example 1, except that Compound a-9 (30 mg, 0.108 mmol) was used instead of Compound a-1.

¹H NMR (400 MHz, DMSO-*d₆*) δ 12.29 (br s, 1H), 7.82 (s, 1H), 7.71 (d, *J* = 4.0 Hz, 1H), 7.55 (dd, *J* = 8.9, 4.2 Hz, 2H), 7.51 - 7.44 (m, 2H), 7.44 - 7.37 (m, 2H), 7.37 - 7.31 (m, 1H), 7.14 (dd, *J* = 8.9, 4.2 Hz, 2H), 6.51 (s, 1H), 5.16 (d, *J* = 3.6 Hz, 2H); LCMS (ESI), m/z = 376.95 [M+H]⁺.

### Example II: Synthesis of phenyl-pyridine or phenyl-pyrimidine skeleton compound

### [Example 10] Preparation of (Z)-5-((2-(p-tolyl)pyridin-4-yl)methylene)thiazolidine-2,4-dione (Compound 10)

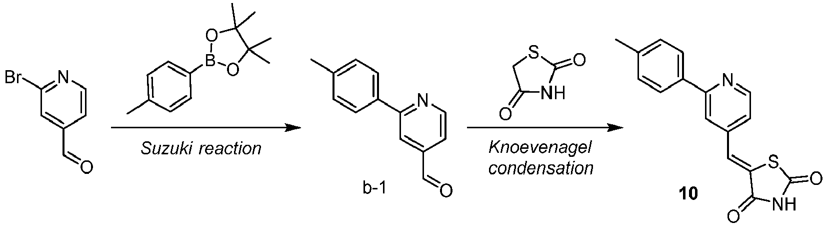

### Step 1: Preparation of 2-(p-tolyl)isonicotinaldehyde (Compound b-1)

2-Bromoisonicotinaldehyde (0.390 g, 2.1 mmol), 4,4,5,5-tetramethyl-2-(*p*-tolyl)-1,3,2-dioxaborolane (0.916 g, 4.2 mmol), and Pd(PPh₃)₄ (0.242 g, 0.210 mmol) were added to toluene (5 ml) under nitrogen, 2 M Na₂CO₃ (5.3 ml) was added dropwise, and stirring was performed at 100°C for 3 hours. After completing the reaction and cooling at room temperature, an organic layer obtained by extraction using EtOAc and water was cooled washed with brine, dried with magnesium sulfate, and filtered. A residue obtained by distilling the filtered solution under reduced pressure was purified by medium pressure liquid chromatography to obtain 2-(*p*-tolyl)isonicotinaldehyde (Compound b-1) (151 mg, yield: 36%) .

¹H NMR (400 MHz, CDCl₃) δ 10.14 (s, 1H), 8.93 (d, *J* = 4.9 Hz, 1H), 8.12 (s, 1H), 8.01 - 7.93 (m, 2H), 7.61 (dd, *J* = 5.0, 1.2 Hz, 1H), 7.36 - 7.29 (m, 2H), 2.43 (s, 3H).

### Step 2: Preparation of (Z)-5-((2-(p-tolyl)pyridin-4-yl)methylene)thiazolidine-2,4-dione (Compound 10)

Compound b-1 (100 mg, 0.507 mmol), thiazolidine-2,4-dione (71.3 mg, 0.608 mmol), and piperidine (60.2 µl, 0.608 mmol) were dissolved in ethanol (3 ml), and reaction was performed by stirring at 100°C for 1.5 hours using a microwave reactor. After completing the reaction, an organic layer obtained by extraction using EtOAc and water was washed with brine, dried with magnesium sulfate, and filtered. A residue obtained by distilling the filtered solution under reduced pressure was purified using medium pressure liquid chromatography (silica gel, 0-50% EtOAc/hexane) to obtain a compound. The obtained compound was recrystallized using EtOAc to obtain a solidified final target compound, (*Z*)-5-((2-(*p*-Tolyl)pyridin-4-yl)methylene)thiazolidine-2,4-dione (Compound 10) (6 mg, yield: 4%).

¹H NMR (400 MHz, MeOD) δ 8.71 (d, *J* = 5.2 Hz, 1H), 7.95 - 7.87 (m, 3H), 7.81 (s, 1H), 7.46 (d, *J* = 5.2 Hz, 1H), 7.36 (d, *J* = 7.9 Hz, 2H), 2.44 (s, 3H); LCMS (ESI), m/z = 297.01 [M+H]⁺.

### [Example 11] Preparation of (Z)-5-((2-(4-(tert-butyl)phenyl)pyridin-4-yl)methylene)thiazolidine-2,4-dione (Compound 11)

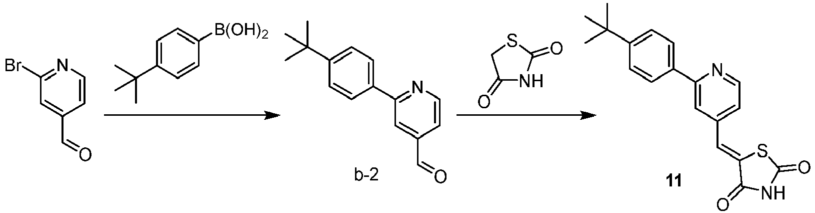

### Step 1: Preparation of 2-(4-(tert-butyl)phenyl)isonicotinaldehyde (Compound b-2)

(2-(4-(*tert*-butyl)phenyl)isonicotinaldehyde) (Compound b-2) was obtained (2.1 g, yield: 82%) in the same manner as in Step 1 of Example 10, except that 4-(*tert-*butyl)phenyl)boronic acid was used instead of 4,4,5,5-tetramethyl-2-(*p*-tolyl)-1,3,2-dioxaborolane.

¹H NMR (400 MHz, CDCl₃) δ 10.14 (s, 1H), 8.93 (d, *J* = 4.9 Hz, 1H), 8.12 (s, 1H), 8.01 (d, *J* = 8.5 Hz, 2H), 7.61 (dd, *J* = 4.9, 1.3 Hz, 1H), 7.54 (d, *J* = 8.5 Hz, 2H), 1.38 (s, 9H).

### Step 2 : Preparation of (Z)-5-((2-(4-(tert-butyl)phenyl)pyridin-4-yl)methylene)thiazolidine-2,4-dione (Compound 11)

The target compound, (*Z*)-5-((2-(4-(*tert-*Butyl)phenyl)pyridin-4-yl)methylene)thiazolidine-2,4-dione (Compound 11), was obtained (159 mg, yield: 27%) in the same manner as in Step 2 of Example 10, except that Compound b-2 (418 mg, 1.747 mmol) was used instead of Compound b-1.

¹H NMR (400 MHz, CDCl₃) δ 8.80 (d, *J* = 5.1 Hz, 1H), 8.43 (br s, 1H), 7.95 (d, *J* = 8.4 Hz, 2H), 7.83 (s, 1H), 7.75 (s, 1H), 7.54 (d, *J* = 8.4 Hz, 2H), 7.26 - 7.23 (m, 1H), 1.38 (s, 9H); LCMS (ESI), m/z = 339.09 [M+H]⁺.

### [Example 12] Preparation of (Z)-5-((2-(4-(trifluoromethyl)phenyl)pyridin-4-yl)methylene)thiazolidine-2,4-dione (Compound 12)

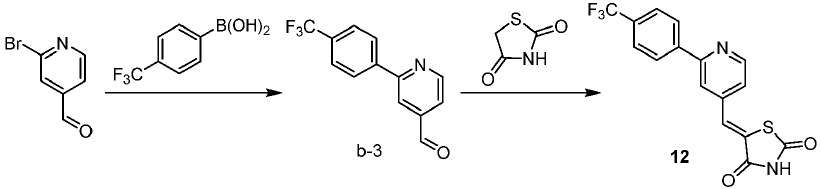

### Step 1: Preparation of 2-(4-(trifluoromethyl)phenyl)isonicotinaldehyde (Compound b-3)

2-(4-(Trifluoromethyl)phenyl) isonicotinaldehyde (Compound b-3) was obtained (219 mg, yield: 41%) in the same manner as in Step 1 of Example 10, except that (4-(trifluoromethyl)phenyl)boronic acid was used instead of 4,4,5,5-tetramethyl-2-(*p*-tolyl)-1,3,2-dioxaborolane.

¹H NMR (400 MHz, CDCl₃) δ 10.17 (s, 1H), 9.00 (d, *J* = 4.8 Hz, 1H), 8.21 (s, 1H), 8.20 - 8.15 (m, 2H), 7.78 (d, *J* = 8.4 Hz, 2H), 7.72 (dd, *J* = 4.8, 1.1 Hz, 1H).

### Step 2: Preparation of (Z)-5-((2-(4-(trifluoromethyl)phenyl)pyridin-4-yl)methylene)thiazolidine-2,4-dione (Compound 12)

The target compound, (*Z*)-5-((2-(4-(trifluoromethyl)phenyl)pyridin-4-yl)methylene)thiazolidine-2,4-dione (Compound 12), was obtained (9 mg, yield: 13%) in the same manner as in Step 2 of Example 10, except that Compound b-3 (50 mg, 0.199 mmol) was used instead of Compound b-1.

¹H NMR (400 MHz, MeOD) δ 8.83 (d, *J* = 5.3 Hz, 1H), 8.23 (d, *J* = 8.2 Hz, 2H), 8.11 (s, 1H), 7.92 - 7.82 (m, 3H), 7.61 (dd, *J* = 5.3, 1.3 Hz, 1H); LCMS (ESI), m/z = 350.95 [M+H]⁺.

### [Example 13] Preparation of (Z)-5-((2-(4-methoxyphenyl)pyridin-4-yl)methylene)thiazolidine-2,4-dione (Compound 13)

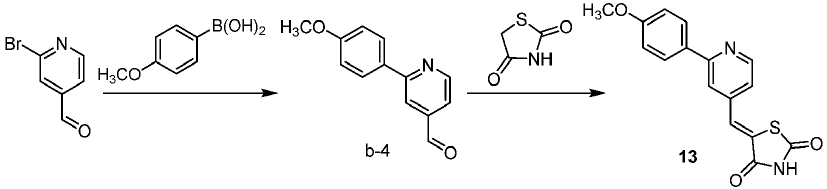

### Step 1: Preparation of 2-(4-methoxyphenyl)isonicotinaldehyde (Compound b-4)

2-(4-Methoxyphenyl)isonicotinaldehyde (Compound b-4) was obtained (364 mg, yield: 79%) in the same manner as in Step 1 of Example 10, except that (4-methoxyphenyl)boronic acid was used instead of 4,4,5,5-tetramethyl-2-(*p*-tolyl)-1,3,2-dioxaborolane.

¹H NMR (400 MHz, CDCl₃) δ 10.13 (s, 1H), 8.90 (d, *J* = 4.9 Hz, 1H), 8.08 (s, 1H), 8.06 - 7.98 (m, 2H), 7.58 (dd, J = 4.9, 1.3 Hz, 1H), 7.08 - 6.97 (m, 2H), 3.89 (s, 3H).

### Step 2: Preparation of (Z)-5-((2-(4-methoxyphenyl)pyridin-4-yl)methylene)thiazolidine-2,4-dione (Compound 13)

The target compound, (*Z*)-5-((2-(4-methoxyphenyl)pyridin-4-yl)methylene)thiazolidine-2,4-dione (Compound 13), was obtained (5 mg, yield: 2%) in the same manner as in Step 2 of Example 10, except that Compound b-4 (140 mg, 0.657 mmol) was used instead of Compound b-1.

¹H NMR (400 MHz, CDCl₃) δ 8.77 (d, *J* = 5.1 Hz, 1H), 8.00 - 7.92 (m, 2H), 7.81 (s, 1H), 7.70 (s, 1H), 7.22 (dd, *J* = 5.2, 1.4 Hz, 1H), 7.06 - 7.00 (m, 2H), 3.89 (s, 3H); LCMS (ESI), m/z = 312.96 [M+H]⁺.

### [Example 14] Preparation of (Z)-5-((2-(4-(trifluoromethoxy)phenyl)pyridin-4-yl)methylene)thiazolidine-2,4-dione (Compound 14)

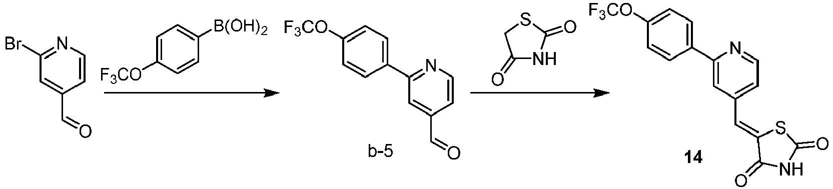

### Step 1: Preparation of 2-(4-(trifluoromethoxy)phenyl)isonicotinaldehyde (Compound b-5)

2-(4-(Trifluoromethoxy)phenyl)isonicotinaldehyde (Compound b-5) was obtained (345 mg, yield: 60%) in the same manner as in Step 1 of Example 10, except that (4-(trifluoromethoxy)phenyl)boronic acid was used instead of 4,4,5,5-tetramethyl-2-(p-tolyl)-1,3,2-dioxaborolane.

¹H NMR (400 MHz, CDCl₃) δ 10.16 (s, 1H), 8.96 (d, *J* = 4.9 Hz, 1H), 8.14 - 8.09 (m, 3H), 7.67 (dd, *J* = 4.9, 1.3 Hz, 1H), 7.40 - 7.32 (m, 2H).

### Step 2: Preparation of (Z)-5-((2-(4-(trifluoromethoxy)phenyl)pyridin-4-yl)methylene)thiazolidine-2,4-dione (Compound 14)

The target compound, (*Z*)-5-((2-(4-(trifluoromethoxy)phenyl)pyridin-4-yl)methylene)thiazolidine-2,4-dione (Compound 14), was obtained (11 mg, yield: 11%) in the same manner as in Step 2 of Example 10, except that Compound b-5 (75 mg, 0.281 mmol) was used instead of Compound b-1.

¹H NMR (400 MHz, CDCl₃) δ 8.91 (br s, 1H), 8.83 (d, *J* = 5.1 Hz, 1H), 8.09 - 7.99 (m, 2H), 7.83 (s, 1H), 7.74 (s, 1H), 7.36 (d, *J* = 8.1 Hz, 2H), 7.32 (dd, J = 5.1, 1.4 Hz, 1H); LCMS (ESI), m/z = 366.98 [M+H]⁺.

### [Example 15] Preparation of (Z)-5-((2-(4-(benzyloxy)phenyl)pyridin-4-yl)methylene)thiazolidine-2,4-dione (Compound 15)

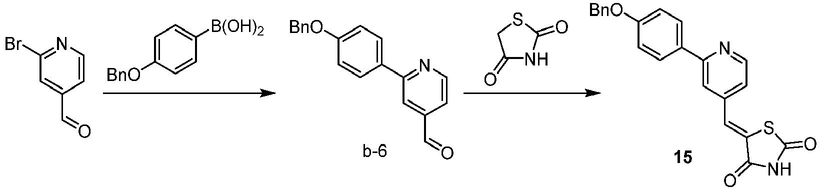

### Step 1: Preparation of 2-(4-(benzyloxy)phenyl)isonicotinaldehyde (Compound b-6)

2-(4-(Benzyloxy)phenyl)isonicotinaldehyde (Compound b-6) was obtained (532 mg, yield: 86%) in the same manner as in Step 1 of Example 10, except that (4-(benzyloxy)phenyl)boronic acid was used instead of 4,4,5,5-tetramethyl-2-(*p*-tolyl)-1,3,2-dioxaborolane.

¹H NMR (400 MHz, CDCl₃) δ 10.07 (s, 1H), 8.86 (d, *J* = 4.9 Hz, 1H), 8.04 - 8.02 (m, 1H), 8.02 - 7.98 (m, 2H), 7.56 - 7.49 (m, 1H), 7.47 - 7.42 (m, 2H), 7.42 - 7.36 (m, 2H), 7.36 - 7.32 (m, 1H), 7.11 - 7.03 (m, 2H), 5.11 (s, 2H).

### Step 2: Preparation of (Z)-5-((2-(4-(benzyloxy)phenyl)pyridin-4-yl)methylene)thiazolidine-2,4-dione (Compound 15)

The target compound, (*Z*)-5-((2-(4-(benzyloxy)phenyl)pyridin-4-yl)methylene)thiazolidine-2,4-dione (Compound 15), was obtained (3 mg, yield: 4.5%) in the same manner as in Step 2 of Example 10, except that Compound b-6 (50 mg, 0.173 mmol) was used instead of Compound b-1.

¹H NMR (400 MHz, CDCl₃) δ 8.77 (d, *J* = 5.1 Hz, 1H), 8.33 (br s, 1H), 7.97 (d, *J* = 8.7 Hz, 2H), 7.81 (s, 1H), 7.70 (s, 1H), 7.51 - 7.31 (m, 5H), 7.22 (dd, *J* = 5.1, 1.0 Hz, 1H), 7.11 (d, *J* = 8.8 Hz, 2H), 5.15 (s, 2H); LCMS (ESI), m/z = 388.98 [M+H]⁺.

### [Example 16] Preparation of (Z)-5-((2-(o-tolyl)pyridin-4-yl)methylene)thiazolidine-2,4-dione (Compound 16)

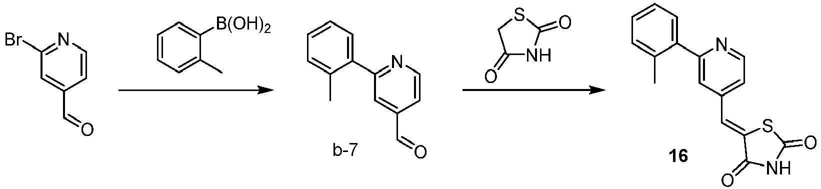

### Step 1: Preparation of 2-(o-tolyl)isonicotinaldehyde (Compound b-7)

2-(o-Tolyl)isonicotinaldehyde (Compound b-7) was obtained (82.5 mg, yield: 78%) in the same manner as in Step 1 of Example 10, except that o-tolylboronic acid was used instead of 4,4,5,5-tetramethyl-2-(*p*-tolyl)-1,3,2-dioxaborolane.

¹H NMR (400 MHz, CDCl₃) δ 10.14 (s, 1H), 8.96 (d, *J* = 4.9 Hz, 1H), 7.82 (s, 1H), 8.01 (dd, *J* = 4.9, 1.4 Hz, 1H), 7.43 (d, *J* = 7.5 Hz, 1H), 7.37 - 7.29 (m, 3H), 2.39 (s, 3H).

### Step 2: Preparation of (Z)-5-((2-(o-tolyl)pyridin-4-yl)methylene)thiazolidine-2,4-dione (Compound 16)

The target compound, (*Z*)-5-((2-(o-tolyl)pyridin-4-yl)methylene)thiazolidine-2,4-dione (Compound 16), was obtained (5.0 mg, yield: 17%) in the same manner as in Step 2 of Example 10, except that Compound b-7 (20 mg, 0.101 mmol) was used instead of Compound b-1.

¹H NMR (400 MHz, MeOD) δ 8.72 (d, *J* = 5.2 Hz, 1H), 7.81 (s, 1H), 7.59 (s, 1H), 7.53 (d, *J* = 4.9 Hz, 1H), 7.38 - 7.31 (m, 4H), 2.33 (s, 3H); LCMS (ESI), m/z = 297.00 [M+H]⁺.

### [Example 17] Preparation of (Z)-5-((2-(m-tolyl)pyridin-4-yl)methylene)thiazolidine-2,4-dione (Compound 17)

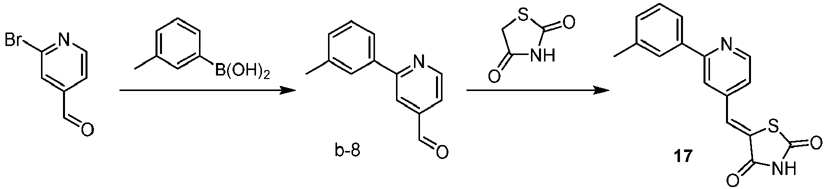

### Step 1: Preparation of 2-(m-tolyl) isonicotinaldehyde (Compound b-8)

2-(*m*-Tolyl)isonicotinaldehyde (Compound b-8) was obtained (97.8 mg, yield: 92%) in the same manner as in Step 1 of Example 10, except that *m*-tolylboronic acid was used instead of 4,4,5,5-tetramethyl-2-(*p*-tolyl)-1,3,2-dioxaborolane.

¹H NMR (400 MHz, CDCl₃) δ 10.15 (s, 1H), 8.94 (d, *J* = 4.8 Hz, 1H), 8.13 (s, 1H), 7.90 (s, 1H), 7.84 (d, *J* = 7.8 Hz, 1H), 7.63 (dd, *J* = 4.9, 1.4 Hz, 1H), 7.40 (t, *J* = 7.6 Hz, 1H), 7.29 (d, *J =* 7.6 Hz, 1H), 2.46 (s, 3H).

### Step 2: Preparation of (Z)-5-((2-(m-tolyl)pyridin-4-yl)methylene)thiazolidine-2,4-dione (Compound 17)

The target compound, (*Z*)-5-((2-(*m*-tolyl)pyridin-4-yl)methylene)thiazolidine-2,4-dione (Compound 17), was obtained (8.1 mg, yield: 18%) in the same manner as in Step 2 of Example 10, except that Compound b-8 (30 mg, 0.152 mmol) was used instead of Compound b-1.

¹H NMR (400 MHz, MeOD) δ 8.70 (d, *J* = 5.2 Hz, 1H), 7.91 (s, 1H), 7.81 (s, 1H), 7.78 (s, 1H), 7.76 (d, *J* = 8.0 Hz, 1H), 7.46 (dd, *J* = 5.2, 1.4 Hz, 1H), 7.40 (t, *J* = 7.6 Hz, 1H), 7.30 (d, *J* = 7.5 Hz, 1H), 2.45 (s, 3H); LCMS (ESI), m/z = 297.00 [M+H]⁺.

### [Example 18] Preparation of (Z)-5-((2-(3-(tert-butyl)phenyl)pyridin-4-yl)methylene)thiazolidine-2,4-dione (Compound 18)

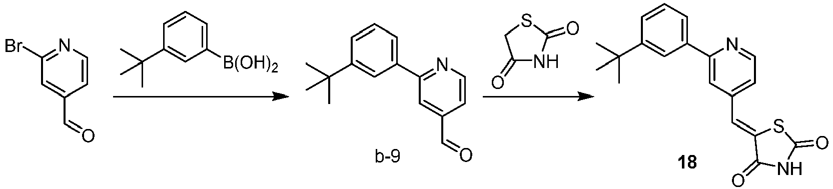

### Step 1: Preparation of 2-(3-(tert-butyl)phenyl)isonicotinaldehyde (Compound b-9)

2-(3-(*tert*-Butyl)phenyl)isonicotinaldehyde (Compound b-9) was obtained (135 mg, yield: 99%) in the same manner as in Step 1 of Example 10, except that (3-(*tert-*butyl)phenyl)boronic acid was used instead of 4,4,5,5-tetramethyl-2-(*p*-tolyl)-1,3,2-dioxaborolane.

¹H NMR (400 MHz, CDCl₃) δ 10.16 (s, 1H), 8.95 (d, *J* = 4.8 Hz, 1H), 8.12 (s, 1H), 8.10 (t, *J* = 1.8 Hz, 1H), 7.84 (dt, *J* = 7.5, 1.4 Hz, 1H), 7.63 (dd, *J* = 4.9, 1.4 Hz, 1H), 7.53 - 7.51 (m, 1H), 7.45 (t, *J* = 7.7 Hz, 1H), 1.41 (s, 9H).

### Step 2: Preparation of (Z)-5-((2-(3-(tert-butyl)phenyl)pyridin-4-yl)methylene)thiazolidine-2,4-dione (Compound 18)

The target compound, (*Z*)-5-((2-(3-(*tert-*butyl)phenyl)pyridin-4-yl)methylene)thiazolidine-2,4-dione (Compound 18), was obtained (7.0 mg, yield: 17%) in the same manner as in Step 2 of Example 10, except that Compound b-9 (30 mg, 0.125 mmol) was used instead of Compound b-1.

¹H NMR (400 MHz, MeOD) δ 8.72 (d, *J* = 5.2 Hz, 1H), 8.05 (s, 1H), 7.92 (s, 1H), 7.83 (s, 1H), 7.76 (d, *J* = 7.7 Hz, 1H), 7.55 (d, *J* = 8.4 Hz, 1H), 7.48 - 7.43 (m, 2H), 1.40 (s, 9H); LCMS (ESI), m/z = 339.02 [M+H]⁺.

### [Example 19] Preparation of (Z)-5-((2-(3,5-Dimethylphenyl)pyridin-4-yl)methylene)thiazolidine-2,4-dione (Compound 19)

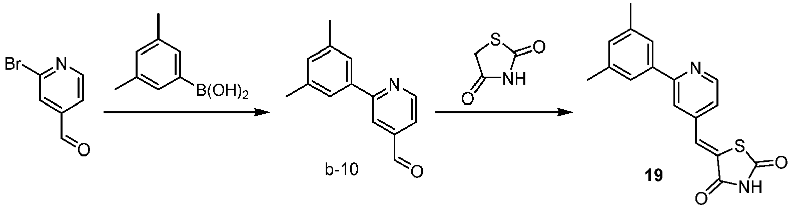

### Step 1: Preparation of 2-(3,5-Dimethylphenyl)isonicotinaldehyde (Compound b-10)

2-(3,5-Dimethylphenyl)isonicotinaldehyde (Compound b-10) was obtained (247.5 mg, yield: 44%) in the same manner as in Step 1 of Example 10, except that 3,5-dimethylphenylboronic acid was used instead of 4,4,5,5-tetramethyl-2-(*p*-tolyl)-1,3,2-dioxaborolane.

¹H NMR (400 MHz, CDCl₃) δ 10.15 (s, 1H), 8.93 (d, *J* = 4.9 Hz, 1H), 8.12 (s, 1H), 7.68 (s, 2H), 7.62 (dd, *J* = 4.9, 1.3 Hz, 1H), 7.12 (s, 1H), 2.42 (s, 6H).

### Step 2: Preparation of (Z)-5-((2-(3,5-Dimethylphenyl)pyridin-4-yl)methylene)thiazolidine-2,4-dione (Compound 19)

The target compound, (*Z*)-5-((2-(3,5-dimethylphenyl)pyridin-4-yl)methylene)thiazolidine-2,4-dione (Compound 19), was obtained (16.5 mg, yield: 22%) in the same manner as in Step 2 of Example 10, except that Compound b-10 (50 mg, 0.237 mmol) was used instead of Compound b-1.

¹H NMR (400 MHz, MeOD) δ 8.61 (d, *J* = 5.3 Hz, 1H), 7.88 (s, 1H), 7.55 (s, 2H), 7.50 (s, 1H), 7.46 (dd, *J* = 5.3, 1.4 Hz, 1H), 7.12 (s, 1H), 2.40 (s, 6H); LCMS (ESI), m/z = 311.02 [M+H]⁺.

### [Example 20] Preparation of 5-((2-(4-(tert-Butyl)phenyl)pyridin-4-yl)methyl)thiazolidine-2,4-dione (Compound 20)

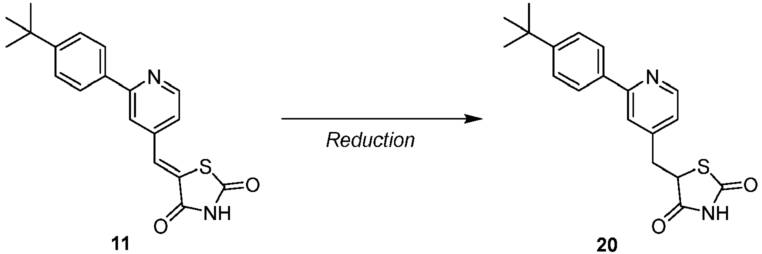

Compound 11 (50 mg, 0.148 mmol) was dissolved in ethanol (3 ml), and Pd/C (31.4 mg, 0.030 mmol) and 37% HCl (0.121 ml, 1.477 mmol) were added dropwise. The reaction mixture was stirred in the conditions of 75°C for 12 hours under hydrogen gas. After completing the reaction, filtration was performed using a filter syringe, and the filtrate was distilled under reduced pressure to obtain a residue. The residue was purified using medium pressure liquid chromatography (silica gel, 0~50% EtOAc/Hexane) to obtain the target compound, 5-((2-(4-(*tert-*butyl)phenyl)pyridin-4-yl)methyl)thiazolidine-2,4-dione (Compound 20) (8 mg, yield: 16%).

¹H NMR (400 MHz, CDCl₃) δ 8.66 (d, *J* = 5.1 Hz, 1H), 7.90 (d, *J* = 8.5 Hz, 2H), 7.59 (s, 1H), 7.51 (d, *J* = 8.5 Hz, 2H), 7.12 (dd, *J* = 5.1, 1.4 Hz, 1H), 4.59 (dd, *J* = 9.4, 4.0 Hz, 1H), 3.56 (dd, *J* = 14.1, 4.0 Hz, 1H), 3.25 (dd, *J* = 14.1, 9.4 Hz, 1H), 1.36 (s, 9H); LCMS (ESI), m/z = 341.05 [M+H]⁺.

### [Example 21] Preparation of (Z)-5-((2-(4-(tert-Butyl)phenyl)pyridin-4-yl)methylene)-3-methylthiazolidine-2,4-dione (Compound 21)

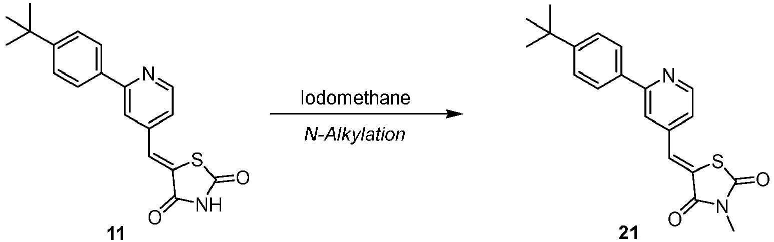

Compound 11 (10 mg, 0.030 mmol) and K₂CO₃ (4.9 mg, 0.035 mmol) were dissolved in DMF (0.3 ml), iodomethane (2.2 µl, 0.035 mmol) was added, and stirring was performed for 4 hours. When the reaction was completed, extraction was performed using EtOAc and water, the obtained organic layer was washed with water and brine, drying was performed with anhydrous magnesium sulfate, and filtration was performed. A residue obtained by distilling the filtrate under reduced pressure was purified by medium pressure liquid chromatography (silica gel, 0~50% EtOAc/Hexane) to obtain the target compound, (*Z*)-5-((2-(4-(*tert*-butyl)phenyl)pyridin-4-yl)methylene)-3-methylthiazolidine-2,4-dione (Compound 21) (6 mg, yield: 58%).

¹H NMR (400 MHz, CDCl₃) δ 8.79 (d, *J* = 5.1 Hz, 1H), 7.95 (d, *J* = 8.5 Hz, 2H), 7.87 (s, 1H), 7.76 (s, 1H), 7.53 (d, *J* = 8.5 Hz, 2H), 7.26 - 7.24 (m, 1H), 3.28 (s, 3H), 1.38 (s, 9H); LCMS (ESI), m/z = 353.08 [M+H]⁺.

### [Example 22] Preparation of (Z)-5-((2-(4-(tert-Butyl)phenyl)pyridin-4-yl)methylene)-3-(2-morpholinoethyl)thiazolidine-2,4-dione (Compound 22)

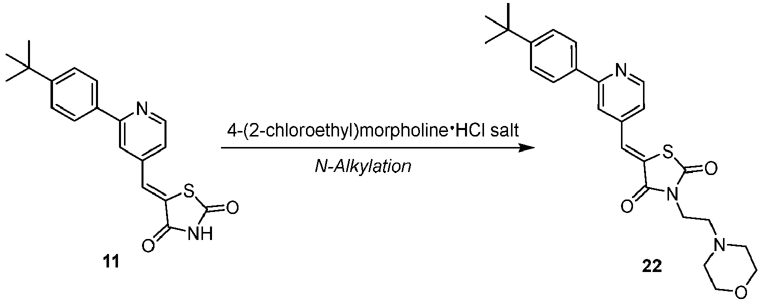

The target compound, (*Z*)-5-((2-(4-(*tert-*butyl)phenyl)pyridin-4-yl)methylene)-3-(2-morpholinoethyl)thiazolidine-2,4-dione (Compound 22), was obtained (10 mg, yield: 38%) in the same manner as in Example 21, except that a 4-(2-chloroethyl)morpholine·HCl salt was used instead of iodomethane.

¹H NMR (400 MHz, CDCl₃) δ 8.79 (d, *J* = 5.1 Hz, 1H), 7.95 (d, *J* = 8.5 Hz, 2H), 7.85 (s, 1H), 7.77 (s, 1H), 7.53 (d, *J* = 8.5 Hz, 2H), 7.30 - 7.24 (m, 1H), 3.91 (t, *J* = 6.3 Hz, 2H), 3.72 - 3.58 (m, 4H), 2.64 (t, *J* = 6.3 Hz, 2H), 2.56 - 2.45 (m, 4H), 1.38 (s, 9H); LCMS (ESI), m/z = 452.22 [M+H]⁺.

### [Example 23] Preparation of (Z)-5-((6-Phenylpyridin-3-yl)methylene)thiazolidine-2,4-dione (Compound 23)

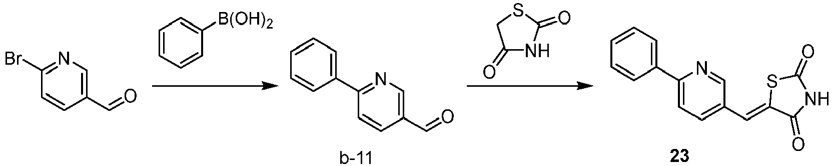

### Step 1: Preparation of 6-Phenylnicotinaldehyde (Compound b-11)

6-Phenylnicotinaldehyde (Compound b-11) was obtained (425 mg, yield: 86%) in the same manner as in Step 1 of Example 10, except that 6-bromonicotinaldehyde and phenylboronic acid was used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.13 (s, 1H), 9.13 (d, *J* = 1.9 Hz, 1H), 8.23 (dd, *J* = 8.3, 2.2 Hz, 1H), 8.09 (dd, *J* = 7.8, 1.8 Hz, 2H), 7.90 (d, *J* = 8.3 Hz, 1H), 7.57 - 7.44 (m, 3H) .

### Step 2: Preparation of (Z)-5-((6-Phenylpyridin-3-yl)methylene)thiazolidine-2,4-dione (Compound 23)

The target compound, (*Z*)-5-((6-phenylpyridin-3-yl)methylene)thiazolidine-2,4-dione (Compound 23), was obtained (10 mg, yield: 16%) in the same manner as in Step 2 of Example 10, except that Compound b-11 (40 mg, 0.218 mmol) was used instead of Compound b-1.

¹H NMR (400 MHz, (CD₃)₂CO) δ 11.35 (br s, 1H), 8.83 (d, *J* = 5.1 Hz, 1H), 8.24 - 8.14 (m, 2H), 8.10 (s, 1H), 7.85 (s, 1H), 7.58 - 7.43 (m, 4H); LCMS (ESI), m/z = 283.01 [M+H]⁺.

### [Example 24] Preparation of (Z)-5-((6-(4-methoxyphenyl)pyridin-3-yl)methylene)thiazolidine-2,4-dione (Compound 24)

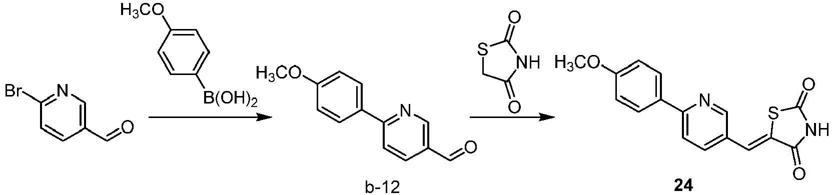

### Step 1: Preparation of 6-(4-Methoxyphenyl)nicotinaldehyde (Compound b-12)

6-(4-Methoxyphenyl)nicotinaldehyde (Compound b-12)was obtained (382 mg, yield: 83%) in the same manner as in Step 1 of Example 10, except that 6-bromonicotinaldehyde and (4-methoxyphenyl)boronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.11 (s, 1H), 9.08 (d, *J* = 1.9 Hz, 1H), 8.19 (dd, *J* = 8.3, 2.1 Hz, 1H), 8.13 - 7.93 (m, 2H), 7.84 (d, *J* = 8.3 Hz, 1H), 7.09 - 6.90 (m, 2H), 3.89 (s, 3H) .

### Step 2: Preparation of (Z)-5-((6-(4-methoxyphenyl)pyridin-3-yl)methylene)thiazolidine-2,4-dione (Compound 24)

The target compound, (*Z*)-5-((6-(4-methoxyphenyl)pyridin-3-yl)methylene)thiazolidine-2,4-dione (Compound 24), was obtained (15.4 mg, yield: 21%) in the same manner as in Step 2 of Example 10, except that Compound b-12 (50 mg, 0.234 mmol) was used instead of Compound b-1.

¹H NMR (400 MHz, DMSO-*d₆*) δ 12.72 (br s, 1H), 8.87 (d, *J* = 2.2 Hz, 1H), 8.14 (d, *J* = 8.9 Hz, 2H), 8.08 (d, *J* = 8.5 Hz, 1H), 7.98 (dd, *J* = 8.5, 2.3 Hz, 1H), 7.85 (s, 1H), 7.08 (d, *J* = 8.9 Hz, 2H), 3.84 (s, 3H); LCMS (ESI), m/z = 313.09 [M+H]⁺.

### [Example 25] Preparation of (Z)-5-((6-(4-(trifluoromethoxy)phenyl)pyridin-3-yl)methylene)thiazolidine-2,4-dione (Compound 25)

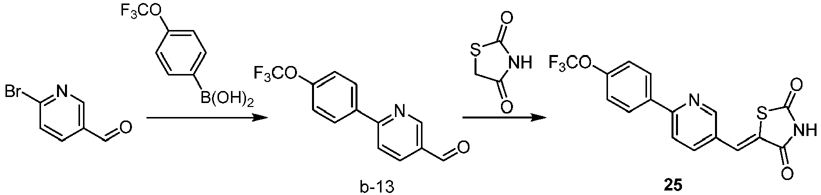

### Step 1: Preparation of 6-(4-(Trifluoromethoxy)phenyl)nicotinaldehyde (Compound b-13)

6-(4-(trifluoromethoxy)phenyl)nicotinaldehyde (Compound b-13) was obtained (332 mg, yield: 58%) in the same manner as in Step 1 of Example 10, except that 6-bromonicotinaldehyde and (4-(trifluoromethoxy)phenyl)boronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.16 (s, 1H), 9.14 (d, *J* = 1.6 Hz, 1H), 8.26 (dd, *J* = 8.2, 2.1 Hz, 1H), 8.19 - 8.07 (m, 2H), 7.90 (d, *J* = 8.2 Hz, 1H), 7.41 - 7.30 (m, 2H).

### Step 2: Preparation of (Z)-5-((6-(4-(trifluoromethoxy)phenyl)pyridin-3-yl)methylene)thiazolidine-2,4-dione (Compound 25)

The target compound, (*Z*)-5-((6-(4-(trifluoromethoxy)phenyl)pyridin-3-yl)methylene)thiazolidine-2,4-dione (Compound 25), was obtained (15 mg, yield: 21%) in the same manner as in Step 2 of Example 10, except that Compound b-13 (50 mg, 0.187 mmol) was used instead of Compound b-1.

¹H NMR (400 MHz, DMSO-*d₆*) δ 8.91 (d, *J* = 2.1 Hz, 1H), 8.28 (d, *J* = 8.9 Hz, 2H), 8.16 (d, *J* = 8.4 Hz, 1H), 8.04 (dd, *J* = *8.5,* 2.2 Hz, 1H), 7.72 (s, 1H), 7.51 (d, *J* = 8.2 Hz, 2H); LCMS (ESI), m/z = 366.99 [M+H]⁺.

### [Example 26] Preparation of (Z)-5-((5-Phenylpyridin-3-yl)methylene)thiazolidine-2,4-dione (Compound 26)

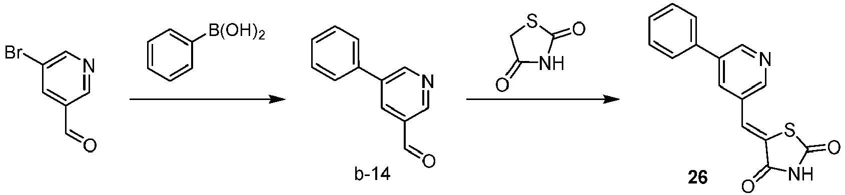

### Step 1: Preparation of 5-Phenylnicotinaldehyde (Compound b-14)

5-Phenylnicotinaldehyde (Compound b-14) was obtained (430 mg, yield: 87%) in the same manner as in Step 1 of Example 10, except that 5-bromonicotinaldehyde and phenylboronic acid was used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.19 (s, 1H), 9.08 (dd, *J* = 12.8, 1.9 Hz, 2H), 8.36 (t, *J* = 2.1 Hz, 1H), 7.66 - 7.59 (m, 2H), 7.55 - 7.38 (m, 3H).

### Step 2: Preparation of (Z)-5-((5-Phenylpyridin-3-yl)methylene)thiazolidine-2,4-dione (Compound 26)

The target compound, (*Z*)-5-((5-phenylpyridin-3-yl)methylene)thiazolidine-2,4-dione (Compound 26), was obtained (16 mg, yield: 21%) in the same manner as in Step 2 of Example 10, except that Compound b-14 (50 mg, 0.273 mmol) was used instead of Compound b-1.

¹H NMR (400 MHz, DMSO-*d₆*) δ 8.95 (d, *J* = 2.1 Hz, 1H), 8.80 (d, *J* = 2.0 Hz, 1H), 8.20 (t, *J* = 2.1 Hz, 1H), 7.91 (s, 1H), 7.81 - 7.75 (m, 2H), 7.55 (t, *J* = 7.4 Hz, 2H), 7.52 - 7.43 (m, 1H); LCMS (ESI), m/z = 282.95 [M+H]⁺.

### [Example 27] Preparation of (Z)-5-((5-(p-tolyl)pyridin-3-yl)methylene)thiazolidine-2,4-dione (Compound 27)

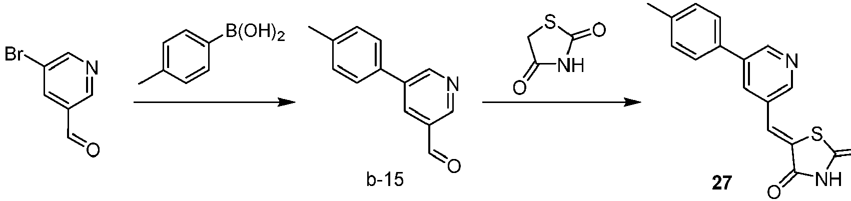

### Step 1: Preparation of 5-(p-tolyl)nicotinaldehyde (Compound b-15)

5-(*p*-tolyl)nicotinaldehyde (Compound b-15) was obtained (468 mg, yield: 88%) in the same manner as in Step 1 of Example 10, except that 5-bromonicotinaldehyde and *p-*tolylboronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.19 (s, 1H), 9.05 (d, *J* = 2.3 Hz, 1H), 9.00 (d, *J* = 1.8 Hz, 1H), 8.31 (t, *J* = 2.1 Hz, 1H), 7.58 (d, *J* = 8.7 Hz, 2H), 7.05 (d, *J* = 8.7 Hz, 2H), 3.88 (s, 3H).

### Step 2: Preparation of (Z)-5-((5-(p-tolyl)pyridin-3-yl)methylene)thiazolidine-2,4-dione (Compound 27)

The target compound, (*Z*)-5-((5-(*p*-tolyl)pyridin-3-yl)methylene)thiazolidine-2,4-dione (Compound 27), was obtained (33 mg, yield: 45%) in the same manner as in Step 2 of Example 10, except that Compound b-15 (50 mg, 0.254 mmol) was used instead of Compound b-1.

¹H NMR (400 MHz, MeOD) δ 8.82 (d, *J* = 2.1 Hz, 1H), 8.70 (d, *J* = 2.0 Hz, 1H), 8.16 (t, *J* = 2.1 Hz, 1H), 7.89 (s, 1H), 7.61 (d, *J* = 8.2 Hz, 2H), 7.36 (d, *J* = 8.0 Hz, 2H), 2.42 (s, 3H); LCMS (ESI), m/z = 296.99 [M+H]⁺.

### [Example 28] Preparation of (Z)-5-((5-(4-(tert-butyl)phenyl)pyridin-3-yl)methylene)thiazolidine-2,4-dione (Compound 28)

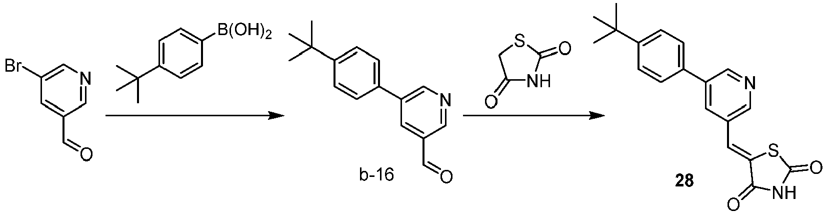

### Step 1: Preparation of 5-(4-(tert-butyl)phenyl)nicotinaldehyde (Compound b-16)

5-(4-(*tert*-butyl)phenyl)nicotinaldehyde (Compound b-16) was obtained (689 mg, yield: 99%) in the same manner as in Step 1 of Example 10, except that 5-bromonicotinaldehyde and 4-(*tert*-butyl)phenyl)boronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.19 (s, 1H), 9.08 (d, *J* = 2.2 Hz, 1H), 9.03 (d, *J* = 1.8 Hz, 1H), 8.34 (t, *J* = 2.1 Hz, 1H), 7.60 - 7.53 (m, 4H), 1.38 (s, 9H).

### Step 2 : Preparation of (Z)-5-((5-(4-(tert-butyl)phenyl)pyridin-3-yl)methylene)thiazolidine-2,4-dione (Compound 28)

The target compound, (*Z*)-5-((5-(4-(*tert-*butyl)phenyl)pyridin-3-yl)methylene)thiazolidine-2,4-dione (Compound 28), was obtained (1.9 mg, yield: 3%) in the same manner as in Step 2 of Example 10, except that Compound b-16 (50 mg, 0.209 mmol) was used instead of Compound b-1.

¹H NMR (400 MHz, MeOD) δ 8.82 (d, *J* = 2.0 Hz, 1H), 8.70 (d, *J* = 2.0 Hz, 1H), 8.18 (t, *J* = 2.0 Hz, 1H), 7.85 (s, 1H), 7.65 (d, *J* = 8.5 Hz, 2H), 7.59 (d, *J* = 8.5 Hz, 2H), 1.38 (s, 9H); LCMS (ESI), m/z = 338.93 [M+H]⁺.

### [Example 29] Preparation of (Z)-5-((4-Phenylpyridin-2-yl)methylene)thiazolidine-2,4-dione (Compound 29)

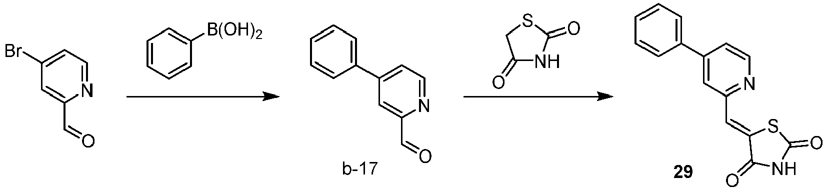

### Step 1: Preparation of 4-phenylpicolinaldehyde (Compound b-17)

4-Phenylpicolinaldehyde (Compound b-17) was obtained (361 mg, yield: 73%) in the same manner as in Step 1 of Example 10, except that 4-bromopicolinaldehyde and phenylboronic acid were used instead of 2-Bromoisonicotinaldehyde and 4,4,5,5-tetramethyl-2-(*p-*tolyl)-1,3,2-dioxaborolane.

### Step 2: Preparation of (Z)-5-((4-Phenylpyridin-2-yl)methylene)thiazolidine-2,4-dione (Compound 29)

The target compound, (*Z*)-5-((4-phenylpyridin-2-yl)methylene)thiazolidine-2,4-dione (Compound 29), was obtained (30 mg, yield: 38%) in the same manner as in Step 2 of Example 10, except that Compound b-17 (50 mg, 0.273 mmol) was used instead of Compound b-1.

¹H NMR (400 MHz, DMSO-*d₆*) δ 12.43 (br s, 1H), 8.80 (d, *J* = 5.2 Hz, 1H), 8.25 (s, 1H), 7.90 (s, 1H), 7.87 (d, *J* = 7.5 Hz, 2H), 7.75 (d, *J* = 5.1 Hz, 1H), 7.62 - 7.45 (m, 3H); LCMS (ESI), m/z = 282.90 [M+H]⁺.

### [Example 30] Preparation of (Z)-5-((4-(p-tolyl)pyridin-2-yl)methylene)thiazolidine-2,4-dione (Compound 30)

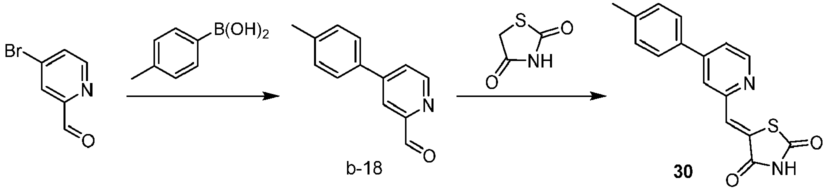

### Step 1: Preparation of 4-(p-tolyl)picolinaldehyde (Compound b-18)

4-(*p*-Tolyl)picolinaldehyde (Compound b-18) was obtained (200 mg, yield: 47%) in the same manner as in Step 1 of Example 10, except that 4-bromopicolinaldehyde and *p-*tolylboronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.14 (s, 1H), 8.80 (d, *J* = 5.1 Hz, 1H), 8.18 (d, *J* = 1.5 Hz, 1H), 7.73 (dd, *J* = 5.1, 1.8 Hz, 1H), 7.60 (d, *J* = 8.1 Hz, 2H), 7.32 (d, *J* = 8.0 Hz, 2H), 2.43 (s, 3H).

### Step 2: Preparation of (Z)-5-((4-(p-tolyl)pyridin-2-yl)methylene)thiazolidine-2,4-dione (Compound 30)

The target compound, (*Z*)-5-((4-(*p*-tolyl)pyridin-2-yl)methylene)thiazolidine-2,4-dione (Compound 30), was obtained (25 mg, yield: 33%) in the same manner as in Step 2 of Example 10, except that Compound b-18 (50 mg, 0.254 mmol) was used instead of Compound b-1.

¹H NMR (400 MHz, DMSO-*d₆*) δ 12.46 (s, 1H), 8.76 (d, *J* = 5.2 Hz, 1H), 8.24 (d, *J* = 1.2 Hz, 1H), 7.89 (s, 1H), 7.77 (d, *J* = 8.2 Hz, 2H), 7.73 (dd, *J* = 5.2, 1.8 Hz, 1H), 7.36 (d, *J* = 8.0 Hz, 2H), 2.37 (s, 3H); LCMS (ESI), m/z = 296.96 [M+H]⁺.

### [Example 31] Preparation of (Z)-5-((4-(4-(tert-butyl)phenyl)pyridin-2-yl)methylene)thiazolidine-2,4-dione (Compound 31)

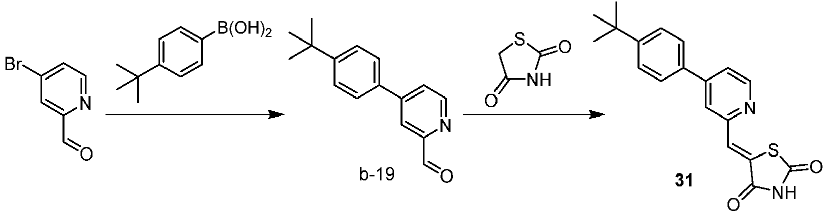

### Step 1: Preparation of 4-(4-(tert-butyl)phenyl)picolinaldehyde (Compound b-19)

(4-(*tert*-Butyl)phenyl)boronic acid (Compound b-19) was obtained (496 mg, yield: 77%) in the same manner as in Step 1 of Example 10, except that 4-bromopicolinaldehyde and 4-(*tert*-butyl)phenyl)boronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.14 (s, 1H), 8.80 (d, *J* = 5.1 Hz, 1H), 8.19 (d, *J* = 1.2 Hz, 1H), 7.73 (dd, *J* = 5.1, 1.9 Hz, 1H), 7.66 - 7.61 (m, 2H), 7.56 - 7.51 (m, 2H), 1.37 (s, 9H).

### Step 2: Preparation of (Z)-5-((4-(4-(tert-butyl)phenyl)pyridin-2-yl)methylene)thiazolidine-2,4-dione (Compound 31)

The target compound, (*Z*)-5-((4-(4-(*tert-*butyl)phenyl)pyridin-2-yl)methylene)thiazolidine-2,4-dione (Compound 31), was obtained (16 mg, yield: 23%) in the same manner as in Step 2 of Example 10, except that Compound b-19 (50 mg, 0.209 mmol) was used instead of Compound b-1.

¹H NMR (400 MHz, DMSO-*d₆*) δ 12.45 (s, 1H), 8.78 (d, *J* = 5.2 Hz, 1H), 8.23 (d, *J* = 1.1 Hz, 1H), 7.90 (s, 1H), 7.79 (d, *J* = 8.5 Hz, 2H), 7.73 (dd, *J* = 5.2, 1.7 Hz, 1H), 7.57 (d, *J* = 8.5 Hz, 2H), 1.33 (s, 9H); LCMS (ESI), m/z = 339.07 [M+H]⁺.

### [Example 32] Preparation of (Z)-5-((4-(4-(trifluoromethyl)phenyl)pyridin-2-yl)methylene)thiazolidine-2,4-dione (Compound 32)

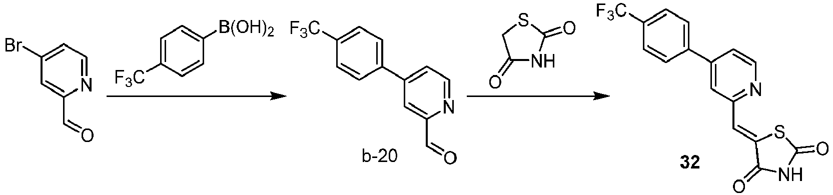

### Step 1: Preparation of 4-(4-(trifluoromethyl)phenyl)picolinaldehyde (Compound b-20)

4-(4-(trifluoromethyl)phenyl)picolinaldehyde (Compound b-20) was obtained (150 mg, yield: 37%) in the same manner as in Step 1 of Example 10, except that 4-bromopicolinaldehyde and (4-(trifluoromethyl)phenyl) boronic acid were used instead of 2-bromoisonicotinaldehyde and 4,4,5,5-tetramethyl-2-(*p*-tolyl)-1,3,2-dioxaborolane.

¹H NMR (400 MHz, CDCl₃) δ 10.16 (s, 1H), 8.89 (d, *J* = 5.0 Hz, 1H), 8.20 (d, *J* = 1.2 Hz, 1H), 7.80 (d, *J* = 2.2 Hz, 3H), 7.77 (d, *J* = 1.8 Hz, 1H), 7.76 (d, *J* = 1.8 Hz, 1H).

### Step 2: Preparation of (Z)-5-((4-(4-(trifluoromethyl)phenyl)pyridin-2-yl)methylene)thiazolidine-2,4-dione (Compound 32)

The target compound, (*Z*)-5-((4-(4-(trifluoromethyl)phenyl)pyridin-2-yl) methylene)thiazolidine-2,4-dione (Compound 32), was obtained (24 mg, yield: 43%) in the same manner as in Step 2 of Example 10, except that Compound b-20 (40 mg, 0.159 mmol) was used instead of Compound b-1.

¹H NMR (400 MHz, DMSO-*d₆*) δ 12.48 (s, 1H), 8.86 (d, *J* = 5.2 Hz, 1H), 8.32 (s, 1H), 8.08 (d, *J* = 8.2 Hz, 2H), 7.93 (d, *J* = 8.6 Hz, 2H), 7.91 (s, 1H), 7.82 (dd, *J* = 5.2, 1.7 Hz, 1H); LCMS (ESI), m/z = 351.00 [M+H]⁺.

### [Example 33] Preparation of (Z)-5-((4-(4-(trifluoromethoxy)phenyl)pyridin-2-yl)methylene)thiazolidine-2,4-dione (Compound 33)

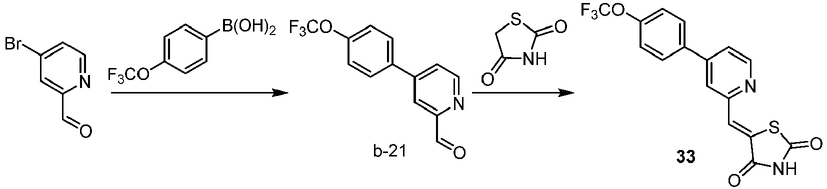

### Step 1: Preparation of 4-(4-(trifluoromethoxy)phenyl)picolinaldehyde (Compound b-21)

4-(4-(Trifluoromethoxy)phenyl)picolinaldehyde (Compound b-21) was obtained (337 mg, yield: 47%) in the same manner as in Step 1 of Example 10, except that 4-bromopicolinaldehyde and (4-(trifluoromethoxy)phenyl)boronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.15 (s, 1H), 8.85 (d, *J* = 5.1 Hz, 1H), 8.21 - 8.14 (m, 1H), 7.77 - 7.70 (m, 3H), 7.37 (d, *J* = 8.1 Hz, 2H).

### Step 2: Preparation of (Z)-5-((4-(4-(trifluoromethoxy)phenyl)pyridin-2-yl)methylene)thiazolidine-2,4-dione (Compound 33)

The target compound, (*Z*)-5-((4-(4-(trifluoromethoxy)phenyl)pyridin-2-yl)methylene)thiazolidine-2,4-dione (Compound 33), was obtained (16 mg, yield: 29%) in the same manner as in Step 2 of Example 10, except that Compound b-21 (40 mg, 0.150 mmol) was used instead of Compound b-1.

¹H NMR (400 MHz, CDCl₃) δ 8.81 (d, *J* = 5.1 Hz, 1H), 8.15 (s, 1H), 7.83 (s, 1H), 7.71 - 7.63 (m, 3H), 7.47 (dd, *J* = 5.1, 1.7 Hz, 1H), 7.37 (d, *J* = 8.2 Hz, 2H); LCMS (ESI), m/z = 367.02 [M+H]⁺.

### [Example 34] Preparation of (Z)-5-((6-(4-(tert-butyl)phenyl)pyrimidin-4-yl)methylene)thiazolidine-2,4-dione (Compound 34)

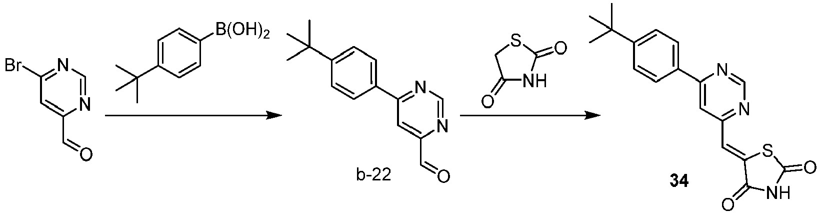

### Step 1: Preparation of 4-(4-(trifluoromethyl)phenyl)picolinaldehyde (Compound b-20)

6-(4-(*tert*-butyl)phenyl)pyrimidine-4-carbaldehyde (Compound b-22) was obtained (26 mg, yield: 41%) in the same manner as in Step 1 of Example 10, except that 6-bromopyrimidine-4-carbaldehyde and 4-(*tert-*butyl)phenyl)boronic acid were used instead of 2-bromoisonicotinaldehyde and 4,4,5,5-tetramethyl-2-(*p-*tolyl)-1,3,2-dioxaborolane.

¹H NMR (400 MHz, CDCl₃) δ 10.11 (s, 1H), 9.50 - 9.41 (m, 1H), 8.26 - 8.19 (m, 1H), 8.17 - 8.07 (m, 2H), 7.61 - 7.53 (m, 2H), 1.38 (s, 9H).

### Step 2 : Preparation of (Z)-5-((6-(4-(tert-butyl)phenyl)pyrimidin-4-yl)methylene)thiazolidine-2,4-dione (Compound 34)

The target compound, (*Z*)-5-((6-(4-(*tert-*butyl)phenyl)pyrimidin-4-yl)methylene)thiazolidine-2,4-dione (Compound 34), was obtained (3 mg, yield: 8%) in the same manner as in Step 2 of Example 10, except that Compound b-22 (26 mg, 0.108 mmol) was used instead of Compound b-1.

¹H NMR (400 MHz, DMSO-*d₆*) δ 12.68 (s, 1H), 9.34 (s, 1H), 8.50 (s, 1H), 8.17 (d, *J* = 8.5 Hz, 2H), 7.81 (s, 1H), 7.61 (d, *J* = 8.5 Hz, 2H), 1.34 (s, 9H); LCMS (ESI), m/z = 340.10 [M+H]⁺.

### <Experimental Example 1> Measurement of activity of derivative using cell based-assay system

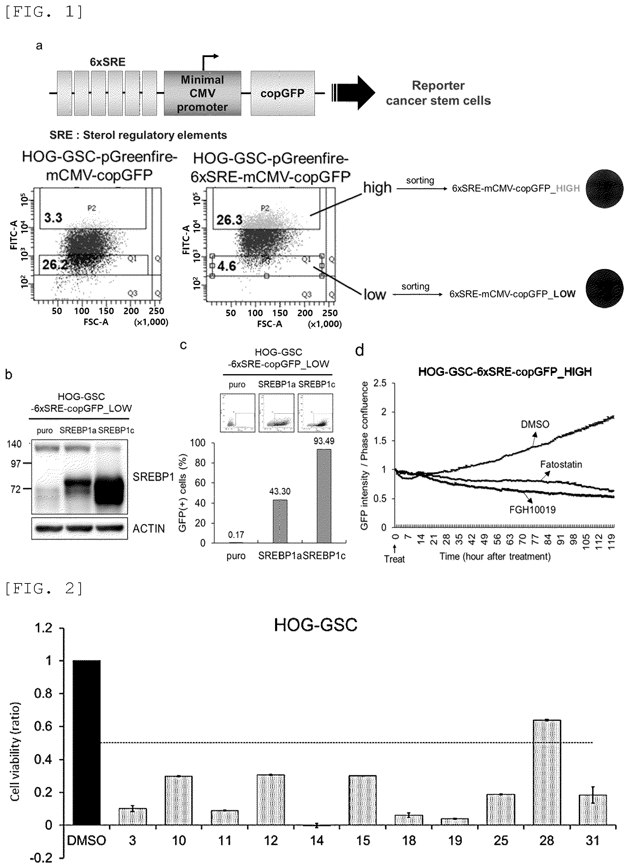

After confirming that a cell-based assay system using a 6xSRE reporter vector (a of FIG. 1) showed copGFP change specific to SREBP protein activity change, the activity of the compound of the present invention was measured as follows.

First, as shown in (a) of FIG. 1, SRE(5'-ATCACCCCAC-3') 6 copies which are DNA sequences to which a transcription factor, SREBP active form bonds were inserted into a minimal CMV promoter 5' front end of a pGreenfire-Lenti-reporter vector to produce a 6xSRE reporter vector. The 6xSRE reporter vector is a vector system in which a minimal CMV promoter is activated only when SREBP in an activated form bonds to 6xSRE and copGFP is expressed, and after the vector was introduced to HOG-GSC which is a brain tumor stem cell, separation into a GFP high cell and a GFP low cell through FACS sorting was performed. Each of SREBP1a and SREBP1c in an active form was overexpressed in separated GFP-low cells (b of FIG. 1), and it was measured therefrom whether the expression amount of GFP was increased. As a result, it was confirmed that the overexpression of SREBP1a and SREBP1c active forms increased GFP expression by 43.30% and 93.49%, in separated GFP-low cells (c of FIG. 1). The separated GFP-high cells were treated with each of fatostatin and FGH10019 known to inhibit activity of DMSO and SREBP which was a control group to measure a change in the expression amount of GFP, and, as a result, it was confirmed that when treated with known materials, fatostatin and FGH10019, the expression of GFP was inhibited by up to 50% (d of FIG. 1).

From the above results, it was confirmed that the cell-based assay system using the 6xSRE reporter vector showed copGFP change specific to SREBP protein activity change, and this was used for the following derivative selection.

HOG-GSC-6xSRE-copGFP_HIGH cells were dispensed into a 96-well plate (Corning) at 5000/well, each well was treated repeatedly 3 times with the compound of the present invention at a concentration of 1 µM after 24 hours, and then all wells were photographed every 3 hours for 72 hours in an incubator (ThermoFisher)at 37%, 5% CO₂, using real-time cell imaging equipment (Incucyte) to photograph changes in copGFP intensity. Thereafter, the intensity of copGFP in which HOG-GSC-6xSRE-copGFP_HIGH cells were treated with DMSO as a control group was set to 1 as a reference point, the activity for each compound was normalized, and the activities of 11 compounds which showed excellent efficacy were normalized and are shown in the following Table A:

**[Table A]**

| Compound No. | Reporter activity at 10 µM |
|---|---|
| 3 | 0.010 |
| 10 | 0.088 |
| 11 | 0.046 |
| 12 | 0.059 |
| 14 | 0.017 |
| 15 | 0.086 |
| 18 | 0.013 |
| 19 | 0.018 |
| 25 | 0.063 |
| 28 | 0.048 |
| 31 | 0.045 |

### <Experimental Example 2> Comparison of cell viability in brain tumor stem cell (MTS assay)

HOG-GSC cells which are brain tumor stem cells were treated with 11 compounds at 10 µM and DMSO as a control group, and the number of surviving cells after 72 hours was evaluated by MTS assay.

HOG-GSC which is a brain tumor stem cell was seeded on a 96-well plate at 5000 cells per well, and was cultured in an incubator (ThermoFisher) at 37°C and 5% CO₂ for 24 hours for stabilization of cells. Thereafter, it was treated with 11 compounds of the present invention at 5 µM each, and treated with the same volume of DMSO as a control group as the volume of each compound. After treatment with the compound of the present invention and the control group, cells were cultured in an incubator at 37°C and 5% CO₂ for 72 hours. Thereafter, since there were many living cells in each well, cell viability was quantified using a cell viability assay kit (LPS solution) of which the color becomes darker with an increase in the number of cells. After treatment with the cell viability assay solution, a reaction was performed in an incubator at 37°C and 5% CO₂ for 3 hours to perform coloration, absorbance at 450 nm was measured using a microplate reader (BioTek), and an absorbance intensity of the control group was set to 1 and the absorbance of cells treated with each compound was normalized and is shown in FIG. 2.

As a result, the compound showing a normalized MTS value of less than 0.5 had a cell viability decreased by 50% or more as compared with the control group, and Compounds 3, 10, 11, 12, 14, 15, 18, 19, 25, 28, and 31 of the present invention were confirmed to have a significant cell viability inhibition effect in brain tumor stem cells (FIG. 2).

### <Experimental Example 3> Western blot assay

HOG-GSC-6xSRE-copGFP_HIGH cells were treated with 5 µM, 2.5 µM, 1.25 µM, 0.625 µM, 0.312 µM, 0.156 µM, and 0.078 µM Compound 11 (Example 11) and DMSO as a control group, respectively, and each cell sample was secured after 24, 48, and 72 hours.

The same amount of protein extracted from each cell sample was developed with 8% SDS-PAGE gel electrophoresis. Proteins developed from SDS-PAGE gel were transferred to a PVDF (Millipore, Billerica, MA, USA) membrane, and an area to which the protein of the membrane was not attached was blocked by reacting with a 5% skim milk-tris-buffered saline 0.1% Tween-20 (TBS-T) solution at room temperature for 1 hour.

As a primary antibody, anti-SREBP1 (BD Bioscience) and anti-SREBP2 (R&D systems) were diluted with a 1% bovine serum albumin (BSA)-TBST solution at 1:1500, anti-ACTIN (Sigma) was diluted with the 1% BSA-TBS-T solution at 1:20000, and the reaction was performed at 4°C for 16 hours.

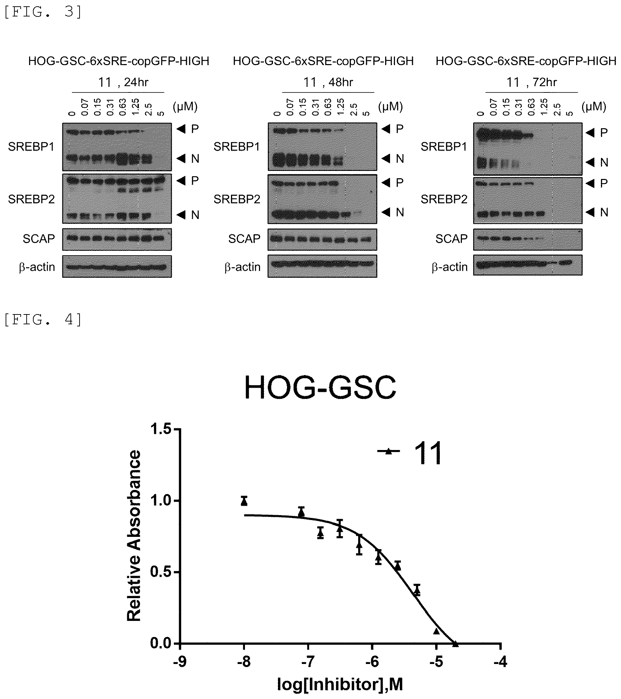

The PVDF membrane was washed with the TBS-T solution at room temperature at 30 rpm for 10 minutes with rocking 3 times, and a goat anti-rabbit IgG HRP (horseradish peroxidase) antibody and a goat anti-mouse IgG HRP secondary antibody were diluted with the 5% skim milk-TBS-T solution at 1:10000, slowly rocked at room temperature and reacted at room temperature for 1 hour. After the membrane was rocked 3 times with TBS-T at room temperature at 30 rpm for 10 minutes to perform washing, luminescence was performed using a HRP substrate, and then imaging was performed using the film. The results are shown in FIG. 3.

### <Experimental Example 4> Cell viability analysis

HOG-GSC which is a brain tumor stem cell was seeded on a 96-well plate at 5000 cells/well, treated with Compound 11 (Example 11) at concentrations of 20 µM, 10 µM, 5 µM, 2.5 µM, 1.25 µM, 0.625 µM, 0.312 µM, 0.156 µM, and 0.078 µM repeatedly 3 times after 24 hours, and treated with the same volume of DMSO as a control group repeatedly 3 times. After the treatment, cells were cultured for 72 hours in an incubator (ThermoFisher) at 37°C, 5% CO₂. Thereafter, since there were many living cells in each well, the cell viability depending on the concentration of Compound 11 (Example 11) was compared using a cell viability assay kit (LPS solution) of which the color becomes darker with an increase in the number of cells. Further, absorbance at 450 nm was measured using a microplate reader (BioTek), and absorbance at each concentration was normalized based on the absorbance intensity of the control group of 1 and is shown in FIG. 4.

A brain tumor stem cell, HOG-GSC was treated with various volumes of Compound 11 (Example 11) and cell viability was measured, and as a result, the cell viability of HOG-GSC was significantly decreased as compared with DMSO as a control group, and the cell viability of HOG-GSC was decreased in proportion to the concentration of Compound 11 (Example 11) (FIG. 4).

It was found therefrom that the compound of the present invention has an excellent apoptosis effect on brain tumor stem cells, and may be used as a brain tumor therapeutic agent targeting brain tumor stem cells.

### <Experimental Example 5> In vivo pharmacokinetics (PK) test and in vivo brain tissue distribution test

### I. In vivo PK test

### 1.1. Experimental drug and excipient

Experimental drug: Compound 11
Excipient: 10% DMSO, 75% PEG400, 15% saline

### 1.2. Experimental system

### mouse, CRljOri:CD1(ICR), SPF 6 weeks old (7 weeks old at the time of administration)

### 1.3. Administration route

oral administration (PO), intravenous administration (IV)

### 1.4. Administration method and number of administration

After obtaining the mouse, the mouse was acclimatized for 5 days, and used in the experiment. An administration dose was 25 mg/kg by oral administration and 5 mg/kg by intravenous administration. A dosage for each individual was calculated based on the weight after fasting (on the day of administration), and the drug was orally administered once to an oral administration (PO) group using a disposable syringe (1 mL) with a zonde for oral administration attached, and intravenously administered to the tail vein of an intravenous administration (IV) group using a disposable syringe (1 mL).

### 1.5. Blood collection and plasma separation

About 0.3 mL of blood was collected from a jugular vein with a 1 mL syringe (25G) treated with 3.8% sodium citrate (about 30-40 ul), placed in one microtube (QSP, 1.5 mL), and centrifuged at 12,000 RPM /3 min to collect plasma. The plasma was frozen and stored at -80°C until analysis, and all animals of a pharmacokinetic experiment group were euthanized after completing the blood collection.

The time of blood collection was as follows.

| Number of animals | Time of blood collection by oral administration (hr) | | | | | | | | | |
|---|---|---|---|---|---|---|---|---|---|---|
| | 0 | 0.25 | 0.5 | 1 | 2 | 4 | 6 | 8 | 24 | Total number of blood collection |
| 4 | V | | | V | | | V | | | 3 |
| 4 | | V | | | V | | | V | | 3 |
| 4 | | | V | | | V | | | V | 3 |

| Number of animals | Time of blood collection by intravenous administration (hr) | | | | | | | | | | | |
|---|---|---|---|---|---|---|---|---|---|---|---|---|
| | 0 | 0.08 | 0.25 | 0.5 | 1 | 2 | 4 | 6 | 8 | 24 | 48 | Total number of blood collection |
| 4 | V | | | | | | | | | | | 1 |
| 4 | | V | | | | | | | | | | 1 |
| 4 | | | V | | | | | | | | | 1 |
| 4 | | | | V | | | | | | | | 1 |
| 4 | | | | | V | | | | | | | 1 |
| 4 | | | | | | V | | | | | | 1 |
| 4 | | | | | | | V | | | | | 1 |
| 4 | | | | | | | | V | | | | 1 |
| 4 | | | | | | | | | V | | | 1 |
| 4 | | | | | | | | | | V | | 1 |
| 4 | | | | | | | | | | | V | 1 |

### 1.6. Concentration analysis and evaluation

### Experiment materials

Mouse blank plasma (Biochemed, 029-APSC-MP-ICR, Sodium citrate)
Internal standard compound: Chlorpropamide (TRC, C424800)

### Sample pretreatment

580 µl of acetonitrile (including internal standard) was added to 20 µl of plasma/tissue, and centrifugation was performed at 4°C at 15,000 rpm for 5 minutes with vortexing. A supernatant obtained after the centrifugation was analyzed with LC-MS/MS.

### 1.7. Result analysis

PK parameter was calculated as a non-compartmental analysis model, using a phoenix WinNonlin 6.4 version (Pharsight, USA) program.

### 1.8. Experiment results

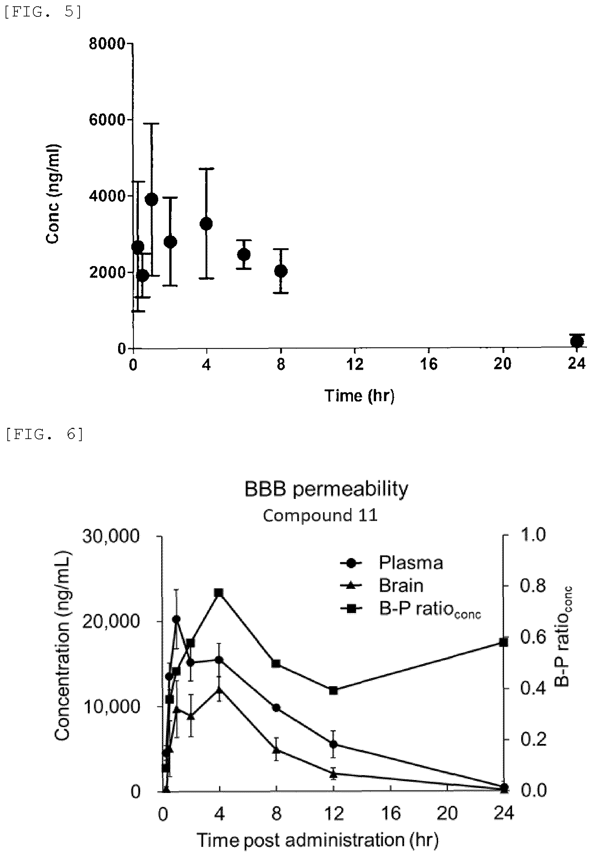

Drug concentration in plasma after oral administration (25 mg/kg) was shown in the following Table 1 and FIG. 5.

**[Table 1]**

| Time (hr) | Individual 1 | Individual 2 | Individual 3 | Individual 4 | Average (ng/ml) | SD |
|---|---|---|---|---|---|---|
| 0.25 | 3210.0 | 2230.0 | 4300.0 | 2000.0 | 2912.5 | 1043.5 |
| 0.5 | 3590.0 | 2340.0 | 4750.0 | 7010.0 | 4422.5 | 1986.0 |
| 1 | 4140.0 | 2380.0 | 2070.0 | 6490.0 | 3770.0 | 2029.6 |
| 2 | 3270.0 | 2030.0 | 4100.0 | 6070.0 | 3867.5 | 1696.9 |
| 4 | 2020.0 | 4420.0 | 2930.0 | 4810.0 | 3545.0 | 1300.0 |
| 6 | 7260.0 | 8180.0 | 2630.0 | 3680.0 | 5437.5 | 2696.5 |
| 8 | 3930.0 | 4120.0 | 4850.0 | 7180.0 | 5020.0 | 1493.6 |
| 24 | 4.1 | BQL | 2.5 | 4.1 | 3.6 | 1.0 |
| 48 | ND | ND | ND | ND | - | - |
| BQL: Below quantifiable limit (< 1 ng/ml) | | | | | | |
| ND: Not detectable | | | | | | |

Drug concentration in plasma after intravenous administration (5 mg/kg) was shown in the following Table 2.

**[Table 2]**

| Time (hr) | Individual 1 | Individual 2 | Individual 3 | Individual 4 | Average (ng/ml) | SD |
|---|---|---|---|---|---|---|
| 0.08 | 2820.0 | 2800.0 | 1900.0 | 2700.0 | 2555.0 | 439.8 |
| 0.25 | 2600.0 | 2240.0 | 2890.0 | 2430.0 | 2540.0 | 275.8 |
| 0.5 | 2790.0 | 1890.0 | 2360.0 | 2000.0 | 2260.0 | 406.4 |
| 1 | 2590.0 | 1780.0 | 2380.0 | 1630.0 | 2095.0 | 462.5 |
| 2 | 1130.0 | 1420.0 | 2240.0 | 1480.0 | 1567.5 | 473.7 |
| 4 | 1050.0 | 1410.0 | 1400.0 | 1260.0 | 1280.0 | 167.9 |
| 6 | 476.0 | 491.0 | 1080.0 | 561.0 | 652.0 | 287.7 |
| 8 | 424.0 | 614.0 | 618.0 | 644.0 | 575.0 | 101.5 |
| 24 | ND | 1.9 | 1.7 | BQL | 1.8 | 0.2 |
| 48 | ND | ND | ND | ND | - | - |
| BQL: Below quantifiable limit (< 1 ng/ml) | | | | | | |
| ND: Not detectable | | | | | | |

When administered at 25 mg/kg by oral administration, it was confirmed that a half-life was 3.9 hours, Tmax was 1.6 hours, Cmax was 5042.5 ng/ml, and AUClast was 39412.0 hr*ng/ml. In addition, when administered at 25 mg/kg by oral administration, bioavailability (BA) was shown to be 62.4% as compared with the case of administration at 5 mg/kg intravenously. Therefore, it was confirmed that the compound according to the present invention showed an in-vivo exposure degree suitable for oral administration.

### II. In vivo Brain tissue distribution test

### 2.1. Experimental drug and excipient

Test drug: Compound 11
Excipient: 20% hydroxypropyl-β-cyclodextrin aqueous solution to which 1% DMSO was added

### 2.2. Experimental system

Mouse, Hsd:ICR(CD-1^{®}), SPF 4 weeks old (7 weeks old at the time of administration)

### 2.3. Administration route

### Oral administration (PO)

### 2.4. Administration method and number of administration

After obtaining the mouse, the mouse was quarantined and acclimatized for 2 weeks, and used in the experiment. An administration dose was 25 mg/kg. A dosage for each individual was calculated based on the weight after fasting for 15 hours (on the day of administration), and the drug was orally administered once using a disposable syringe (1 mL) with a zonde for oral administration attached.

### 2.5. Collection of blood and brain tissue

After respiratory anesthesia, blood was collected by heart blood collection with a 1 mL syringe, placed in a K2-EDTA-treated tube, and centrifuged at 8,000 RPM at 4°C for 20 minutes, and plasma was collected from a supernatant. Heat perfusion was performed on animals after blood collection, a brain tissue was harvested, and olfactory bulb, cerebellum, pones, and medulla oblongata were removed from the harvested brain tissue. The collected plasma and brain tissue were frozen and stored at -80°C until analysis.

The time of blood collection and brain tissue collection were as follows.

| Number of animals | Time of oral administration blood and brain tissue collection (hr) | | | | | | | | |
|---|---|---|---|---|---|---|---|---|---|
| | 0.25 | 0.5 | 1 | 2 | 4 | 8 | 12 | 24 | Total number of collection |
| 6 | V | | | | | | | | 1 |
| 6 | | V | | | | | | | 1 |
| 6 | | | V | | | | | | 1 |
| 6 | | | | V | | | | | 1 |
| 6 | | | | | V | | | | 1 |
| 6 | | | | | | V | | | 1 |
| 6 | | | | | | | V | | 1 |
| 6 | | | | | | | | V | 1 |

In order to secure a sufficient amount of analysis sample, a mixed (pulled) sample of brain tissue and blood secured from two individuals was used (securing three repetitive samples from 6 animals in one collection). Blood plasma and brain tissue were collected from a mouse of the same week of age to which the compound was not administered, and used as a blank blood plasma and a blank tissue in the material concentration analysis.

### 2.6. Concentration analysis and evaluation

### Experiment materials

Internal standard compound: Verapamil hydrochloride (TCI, V0118)

### Sample pretreatment

190 µL of acetonitrile was added to 10 µL of a blood plasma sample including 200 ng/mL of the internal standard material and vortexed at 2,500 RPM for 60 seconds. Thereafter, centrifugation was performed at 12,000 RPM for 5 minutes, and 50 µL of a supernatant was taken and mixed with 50 µL of acetonitrile. 2 µL thereof was injected into LC-MS/MS.

The brain tissue was weighed, third distilled water equivalent to 4 times (w/v = 1/4) the weight was added, and homogenization was performed using a homogenizer. 10 µL of the homogenized tissue mixed solution was taken, 190 µL of acetonitrile including 50 ng/mL of the internal standard material was added, and vortexing was performed at 2,500 RPM for 60 seconds. Thereafter, centrifugation was performed at 12,000 RPM for 10 minutes, and 2 µL of a supernatant was taken and injected into LC-MS/MS.

### 2.7. Result analysis

PK parameter was calculated with a Non-compartmental analysis model using a PKSolver2.0 software.

### 2.8. Experiment results

Concentration in blood and brain tissue after oral administration of Compound 11 (25 mg/kg) is shown in FIG. 6, and the PK parameter calculated therefrom is shown in the following Table 3.

**[Table 3]**

| PK parameter | Plasma (25 mg/kg, PO) | | Brain (25 mg/kg, PO) | |
|---|---|---|---|---|
| | Mean | SD | Mean | SD |
| AUClast (ng/mL x hr) | 176657.416 | 6172.028 | 95996.696 | 5294.400 |
| AUCinf (ng/mL x hr) | 179695.553 | 4955.670 | 96776.970 | 6179.953 |
| MRTinf (hr) | 6.954 | 0.847 | 5.998 | 0.174 |
| Cmax (ng/mL) | 20389.780 | 3277.509 | 12595.041 | 555.226 |
| Tmax (hr) | 1.333 | 0.577 | 3.000 | 1.732 |
| t1/2 (hr) | 3.080 | 1.863 | 2.829 | 1.008 |
| Clobs (mL/min/kg) | 2.320 | 0.065 | - | - |
| Vzobs (L/kg) | 0.617 | 0.369 | - | - |
| Brain-to-plasma ratio | by AUC (brain/plasma) | | 0.543 | |
| | by concentration (brain/plasma) | | Average 0.470; Max 0.777 | |

As a result of analyzing drug concentration in the brain tissue after oral administration, a concentration rate in the brain tissue as compared with that in the blood was confirmed to be 0.470 on average and 0.777 at maximum. A drug exposure degree (area under the curve, AUC) rate in the brain tissue as compared with that in the blood was confirmed to be 0.543.

### <Experimental Example 6> Evaluation of in vivo efficacy

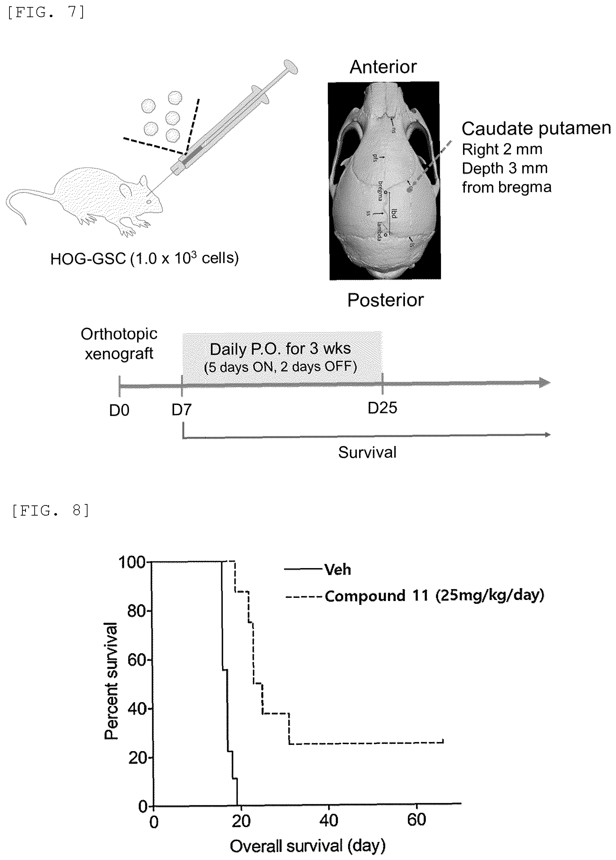

In order to confirm tumor growth inhibition efficacy of the compound of the example according to the present invention in an animal model, the following anticancer efficacy evaluation was performed (FIG. 7).

First, HOG-GSC which is a brain tumor stem cell line was transplanted to a caudate putamen in the brain of a BALB/c nude mouse to establish am orthotropic xenograft model. After transplanting the brain tumor stem cells, the implementation compound of the present invention was orally administered from day 7 for 3 weeks with a schedule of 5 days of administration and 2 days of off medication, and only excipient was administration in the same manner to the control group. Thereafter, based on clinical findings resulting from the growth of the brain tumor in the animals, the survival period of the mouse was investigated, thereby confirming that the death of the animal due to a tumor was delayed by the administration of the implementation compound of the present invention. In addition, both the control mouse and the mouse to which the implementation compound of the present invention was administered were euthanized on day 16 after transplanting the tumor, their brain tissues were harvested, and the sizes of the brain tumor on the same date were compared.

The results of the present anticancer efficacy evaluation are shown in FIGS. 8. 9. and 10.

FIG. 8 is a drawing showing the survival rate of the mouse over time, and it was found that the animal to which the implementation compound of the present invention was administered survived longer than the control animal.

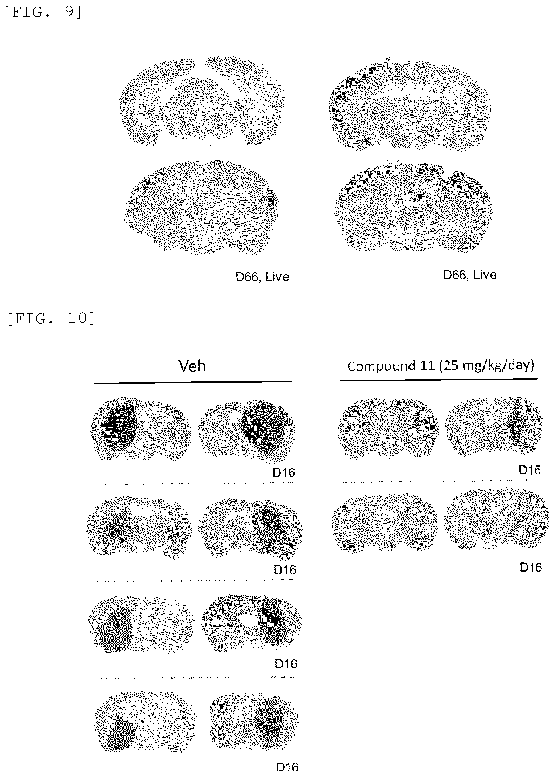

FIG. 9 is a photograph of the brain tissue of the mouse which finally survived on day 66 after tumor transplantation, in which no tumor was observed.

FIG. 10 is a photograph of the brain tissue of the mouse on day 16 after tumor transplantation, and Table 4 quantifies the experimental results of FIG. 10 as a ratio of tumor cross-sectional area to the total brain tissue cross-sectional area. When the implementation compound of the present invention was administered, it was confirmed that the size of the tumor was decreased or the tumor was not found as compared with the control group. Thus, it was found therefrom that the compound according to the present invention has an excellent tumor growth inhibition effect and may be used as a new brain tumor therapeutic agent targeting brain tumor stem cells.

**[Table 4]**

| Individual No. | Tumor cross-sectional area/ brain cross-sectional ratio | |
|---|---|---|
| | Veh | Compound 11 (25 mg/kg/day) |
| 1 | 0.299206 | 0.043726 |
| 2 | 0.165882 | 0 |
| 3 | 0.200111 | - |
| 4 | 0.151627 | - |
| Average | 0.204207 | 0.021863 |

## Claims

1. A substituted thiazolidinedione derivative compound represented by the following Chemical Formula 1, a hydrate thereof, or a pharmaceutically acceptable salt thereof: 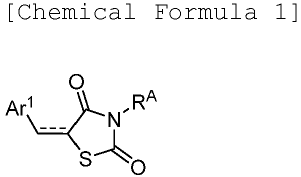 wherein
-̅ -̅ -̅ is a single bond or double bond;
Ar¹ is 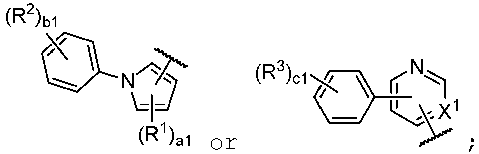
R¹ is C1-C10alkyl;
R² is haloC1-C10alkyloxy or C6-C12arylC1-C10alkyloxy;
R³ is C1-C10alkyl, haloC1-C10alkyl, C1-C10alkoxy, haloC1-C10alkoxy, or C6-C12arylalkyloxy;
X¹ is CH or N;
R^{A} is hydrogen, C1-C10alkyl, C3-C10cycloalkyl, C6-C12aryl, C6-C12arylC1-C10alkyl, or -L¹-Het¹;
L¹ is C1-C10alkylene;
Het¹ is C3-C10heterocycloalkyl;
a1 is an integer of 0 to 3; and
b1 and c1 are independently of each other an integer of 0 to 5.

2. The substituted thiazolidinedione derivative compound, the hydrate thereof, or the pharmaceutically acceptable salt thereof of claim 1,
wherein -̅ -̅ -̅ is a double bond;
Ar¹ is 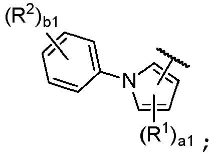
R² is haloC1-C4alkyloxy or C6-C12arylC1-C4alkyloxy;
a1 is an integer of 0 to 2;
b1 is an integer of 0 to 2;
R^{A} is hydrogen, C1-C4alkyl, C3-C8cycloalkyl, or -L¹-Het¹;
L¹ is C1-C4alkylene; and
Het¹ is C3-C8heterocycloalkyl.

3. The substituted thiazolidinedione derivative compound, the hydrate thereof, or the pharmaceutically acceptable salt thereof of claim 1,
wherein Ar¹ is 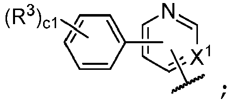
X¹ is CH or N;
R³ is C1-C4alkyl, haloC1-C4alkyl, C1-C4alkoxy, haloC1-C4alkoxy, or C6-C12arylC1-C4alkyloxy;
c1 is an integer of 0 to 2;
R^{A} is hydrogen, C1-C4alkyl, C3-C8cycloalkyl, or -L¹-Het¹;
L¹ is C1-C4alkylene; and
Het¹ is C3-C8heterocycloalkyl.

4. The substituted thiazolidinedione derivative compound, the hydrate thereof, or the pharmaceutically acceptable salt thereof of claim 1, wherein Ar¹ is selected from the following structures:

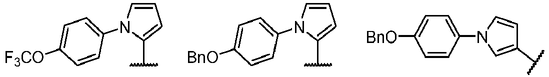

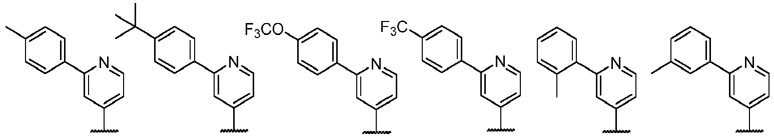

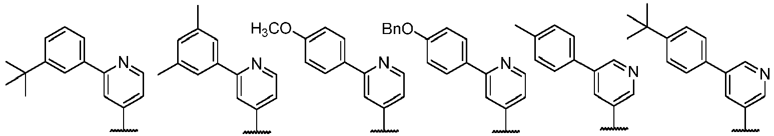

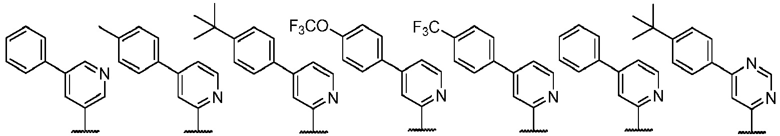

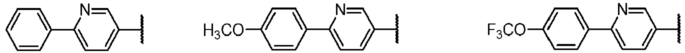

5. The substituted thiazolidinedione derivative compound, the hydrate thereof, or the pharmaceutically acceptable salt thereof of claim 1, wherein R^{A} is hydrogen, methyl, ethyl,

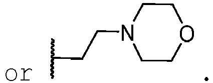

6. The substituted thiazolidinedione derivative compound, the hydrate thereof, or the pharmaceutically acceptable salt thereof of claim 1, wherein the compound represented by Chemical Formula 1 is any one selected from the following compound group:
(*Z*)-5-((1-(4-(trifluoromethoxy)phenyl)-1*H*-pyrrol-2-yl)methylene)thiazolidin-2,4-dione;
(*Z*)-5-((1-(4-(benzyloxy)phenyl)-1*H*-pyrrol-2-yl)methylene)thiazolidin-2,4-dione;
(*Z*)-5-((1-(4-(benzyloxy)phenyl)-1*H*-pyrrol-3-yl)methylene)thiazolidin-2,4-dione;
(*Z*)-5-((2-(*p*-tolyl)pyridin-4-yl)methylene)thiazolidin-2,4-dione;
(*Z*)-5-((2-(4-(*t*-butyl)phenyl)pyridin-4-yl)methylene)thiazolidin-2,4-dione;
(*Z*)-5-((2-(4-(trifluoromethyl)phenyl)pyridin-4-yl)methylene)thiazolidin-2,4-dione;
(*Z*)-5-((2-(4-methoxyphenyl)pyridin-4-yl)methylene)thiazolidin-2,4-dione;
(*Z*)-5-((2-(4-(trifluoromethoxy)phenyl)pyridin-4-yl)methylene)thiazolidin-2,4-dione;
(*Z*)-5-((2-(4-(benzyloxy)phenyl)pyridin-4-yl)methylene)thiazolidin-2,4-dione;
(*Z*)-5-((2-(*o*-tolyl)pyridin-4-yl)methylene)thiazolidin-2,4-dione;
(Z)-5-((2-(*m*-tolyl)pyridin-4-yl)methylene)thiazolidin-2,4-dione;
(Z)-5-((2-(3-(*t*-butyl)phenyl)pyridin-4-yl)methylene)thiazolidin-2,4-dione;
(*Z*)-5-((2-(3,5-dimethylphenyl)pyridin-4-yl)methylene)thiazolidin-2,4-dione;
5-((2-(4-(*t*-butyl)phenyl)pyridin-4-yl)methyl)thiazolidin-2,4-dione;
(*Z*)-5-((2-(4-(*t*-butyl)phenyl)pyridin-4-yl)methylene)-3-methylthiazolidin-2,4-dione;
(*Z*)-5-((2-(4-(*t*-butyl)phenyl)pyridin-4-yl)methylene)-3-(2-morpholinoethyl)thiazolidin-2,4-dione;
(*Z*)-5-((6-phenylpyridin-3-yl)methylene)thiazolidin-2,4-dione;
(*Z*)-5-((6-(4-methoxyphenyl)pyridin-3-yl)methylene)thiazolidin-2,4-dione;
(*Z*)-5-((6-(4-(trifluoromethoxy)phenyl)pyridin-3-yl)methylene)thiazolidin-2,4-dione;
(*Z*)-5-((5-phenylpyridin-3-yl)methylene)thiazolidin-2,4-dione;
(*Z*)-5-((5-(*p*-tolyl)pyridin-3-yl)methylene)thiazolidin-2,4-dione;
(*Z*)-5-((5-(4-(*t*-butyl)phenyl)pyridin-3-yl)methylene)thiazolidin-2,4-dione;
(*Z*)-5-((4-phenylpyridin-2-yl)methylene)thiazolidin-2,4-dione;
(*Z*)-5-((4-(*p*-tolyl)pyridin-2-yl)methylene)thiazolidin-2,4-dione;
(*Z*)-5-((4-(4-(*t*-butyl)phenyl)pyridin-2-yl)methylene)thiazolidin-2,4-dione;
(*Z*)-5-((4-(4-(trifluoromethyl)phenyl)pyridin-2-yl)methylene)thiazolidin-2,4-dione;
(*Z*)-5-((4-(4-(trifluoromethoxy)phenyl)pyridin-2-yl)methylene)thiazolidin-2,4-dione; and
(*Z*)-5-((6-(4-(*t*-butyl)phenyl)pyrimidin-4-yl)methylene)thiazolidin-2,4-dione.

7. A pharmaceutical composition for preventing or treating cancer comprising a substituted thiazolidinedione derivative compound represented by the following Chemical Formula 2, a hydrate thereof, a pharmaceutically acceptable salt thereof: 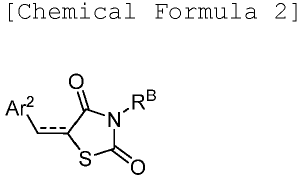 wherein
-̅ -̅ -̅ is a single bond or double bond;
Ar² is 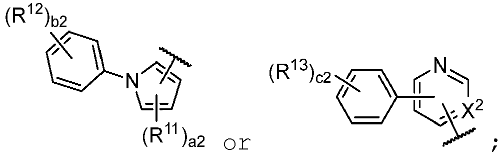
R¹¹ is C1-C10alkyl;
R¹² is C1-C10alkyl, hydroxy, haloC1-C10alkyloxy, or C6-C12arylC1-C10alkyloxy;
R¹³ is C1-C10alkyl, haloC1-C10alkyl, C1-C10alkoxy, haloC1-C10alkoxy, or C6-C12arylalkyloxy;
X² is CH or N;
R^{B} is hydrogen, C1-C10alkyl, C3-C10cycloalkyl, C6-C12aryl, C6-C12arylC1-C10alkyl, or -L²-Het²;
L² is C1-C10alkylene;
Het² is C3-C10heterocycloalkyl;
a2 is an integer of 0 to 3; and
b2 and c2 are independently of each other an integer of 0 to 5.

8. The pharmaceutical composition of claim 7, wherein the cancer is a brain tumor.

9. The pharmaceutical composition of claim 7, wherein the pharmaceutical composition selectively inhibits sterol regulatory element-binding protein-1 (SREBP1).

10. A health functional food composition for preventing or ameliorating cancer comprising a substituted thiazolidinedione derivative compound represented by the following Chemical Formula 2, a hydrate thereof, a pharmaceutically acceptable salt thereof: 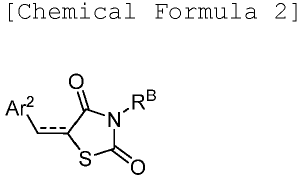 wherein
-̅ -̅ -̅ is a single bond or double bond;
Ar² is 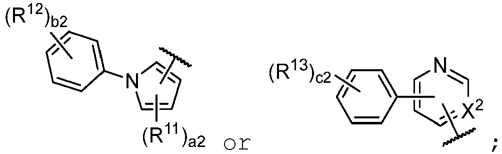
R¹¹ is C1-C10alkyl;
R¹² is C1-C10alkyl, hydroxy, haloC1-C10alkyloxy, or C6-C12arylC1-C10alkyloxy;
R¹³ is C1-C10alkyl, haloC1-C10alkyl, C1-C10alkoxy, haloC1-C10alkoxy, or C6-C12arylalkyloxy;
X² is CH or N;
R^{B} is hydrogen, C1-C10alkyl, C3-C10cycloalkyl, C6-C12aryl, C6-C12arylC1-C10alkyl, or -L²-Het²;
L² is C1-C10alkylene;
Het² is C3-C10heterocycloalkyl;
a2 is an integer of 0 to 3; and
b2 and c2 are independently of each other an integer of 0 to 5.
